# EUROPEAN PATENT SPECIFICATION

(11) **EP 2 044 125 B1**
(45) Date of publication and mention of the grant of the patent: **08.09.2010**
(21) Application number: 07766524.8
(22) Date of filing: 08.06.2007
(51) Int. Cl.: C07K 19/00, C07K 16/32, A61K 39/395, A61P 35/00

(54) **COMPOSITIONS AND METHODS FOR DELIVERING ANTI-ACTIVATED RAS ANTIBODIES INTO CELLS**
ZUSAMMENSETZUNGEN UND VERFAHREN ZUR VERABREICHUNG ANTI-AKTIVIERTER RAS-ANTIKÖRPER AN ZELLEN
COMPOSITIONS ET PROCÉDÉS PERMETTANT D'INTRODUIRE DES ANTICORPS ANTI-RAS ACTIVÉE DANS DES CELLULES

(30) Priority: 12.06.2006 EP 06290952
(43) Date of publication of application: 08.04.2009
(73) Proprietor: CELLECTIS, 93235 Romainville (FR)
(72) Inventor: DE COUPADE, Catherine, 78000 Versailles (FR)
(74) Representative: Novagraaf Technologies
(86) International application number: PCT/IB2007/001546
(87) International publication number: WO 2007/144730

(56) References cited:
- WO-A2-2004/046188
- WO-A2-2005/016960
- US-A1- 2003 199 677
- DE COUPADE CATHERINE ET AL: "Novel human-derived cell-penetrating peptides for specific subcellular delivery of therapeutic biomolecules." THE BIOCHEMICAL JOURNAL. 1 SEP 2005, vol. 390, no. Pt 2, 1 September 2005 (2005-09-01), pages 407-418, XP002404284 ISSN: 1470-8728 cited in the application
- WADIA J S ET AL: "Transmembrane delivery of protein and peptide drugs by TAT-mediated transduction in the treatment of cancer" ADVANCED DRUG DELIVERY REVIEWS, AMSTERDAM, NL, vol. 57, no. 4, 28 February 2005 (2005-02-28), pages 579-596, XP004752189 ISSN: 0169-409X
- FITTIPALDI ANTONIO ET AL: "Transcellular protein transduction using the Tat protein of HIV-1" ADVANCED DRUG DELIVERY REVIEWS, vol. 57, no. 4, 28 February 2005 (2005-02-28), pages 597-608, XP004752190 ISSN: 0169-409X
- TANAKA T ET AL: "INTRABODIES BASED ON INTRACELLULAR CAPTURE FRAMEWORKS THAT BIND THE RAS PROTEIN WITH HIGH AFFINITY AND IMPAIR ONCOGENIC TRANSFORMATION" EMBO JOURNAL, OXFORD UNIVERSITY PRESS, SURREY, GB, vol. 22, no. 5, 3 March 2003 (2003-03-03), pages 1025-1035, XP008028868 ISSN: 0261-4189

## Description

This invention relates generally to compositions and methods of treating proliferative disorders such as cancers and more particularly to compositions comprising an antibody conjugated to a cell-penetrating peptide.

RAS proteins are prototypical G-proteins that have been shown to play a key role in signal transduction, proliferation, and malignant transformation. Activated (oncogenic) RAS-p21 protein has been implicated as a major causative factor in a high proportion of human solid tumors. Mutated *ras* genes have been detected in a large number of cancers comprising adenocarcinoma of the pancreas (90%), the colon (50%) and the lung (30%). Inhibition of Ki-*ras* oncogene expression in human tumor cell lines caused complete regression in animal models (Shirasawa et al., Science 260, 85-88 (1993)). Consequently, the RAS pathway is an attractive target for therapeutic approaches (Downward, Nat. Rev. Cancer. 3(1), 11-22 (2003)).

Antibodies are extremely specific, sometimes far more specific than small-molecule drugs, and are not inherently toxic. Antigen binding analysis can report affinities (K_{D}) close to the low nanomolar range as measured by binding ELISA. This specificity makes monoclonal antibody technology valuable for therapeutic uses. The neutralizing rat monoclonal antibody Y13259 (IgG1) (ATCC number: CRL-1742), which recognizes all four of the mutated mammalian RAS-p21 proteins (H, N, K-4A and K-4B), severely hampers the nucleotide exchange reaction between p21-bound and exogenous guanine nucleotides (Furth et al., J Virol 43, 294-304 (1982); Hattori et al., Mol Cell Biol 7, 1999-2002 (1987)) and has been shown to reverse the phenotype of NIH 3T3 cells transformed by oncogenic *ras* gene after microinjection or elicited tumor regression in nude mice using anti-RAS scFv and an adenoviral vector (Cochet et al., Cancer Res. 58, 1170-1176 (1998) ; Kung et al., Exp. Cell Res. 162, 363-371 (1986)).

Although RAS-p21 protein represents a validated therapeutic target in oncology, therapeutic antibody approaches are not possible because RAS-p21 protein, like many therapeutic targets, is an intracellular protein and because antibodies are unable to cross the cell or nuclear membranes in order to interact with their intracellular antigens. Thus, it is the main object of the invention to provide more effective antibodies and pharmaceutical compositions for treatment of cancer.

PCT patent application n° WO 04/041688 describes immunoglobulin molecules capable of specifically binding to activated RAS protein within an intracellular environment wherein the antibody comprises a heavy chain variable domain and a light chain variable domain. It also discloses that these immunoglobulins can be fused or conjugated to a domain or sequence from a protein responsible for translocation activity (by this means, the immunoglobulin is able to enter the cell or its nucleus when introduced in the vicinity of the cell), such as the domains and sequences from HIV-1-trans-activating protein (Tat), *Drosophila* Antennapedia homeodomain protein and the herpes simplex-1 virus VP22 protein. Also of use for the delivery of antibodies to cells is the TLM peptide (Oess and Hildt, Gene Ther., 7, 750-758, (2000)) or an anti-DNA antibody technology. Use of single domain antibody could have certain limitations including blood stability during *in vivo* studies or low antigen specificity. This shows the importance of an optimal delivery system that could protect the antibody and improve its biodistribution. Delivery peptides reported originate from viral proteins (Tat, VP22, TLM) or non human sequences (*Antennapedia*) and may be both toxic and immunogenic in humans.

Within the framework of research that has led to this invention, the applicant conjugated the rat monoclonal antibody Y13259 (ATCC number: CRL-1742) to certain cell penetrating peptides (CPPs) to enable its translocation inside the cells.

This invention specifically has as its object an anti-activated RAS neutralizing antibody conjugated to at least one cell-penetrating peptide (CPP) for mediating an anti-proliferative activity. Applicant has shown that linking cell-penetrating peptides to monoclonal antibodies enhances their effectiveness against cancer cells.

According to another embodiment, the selected anti-activated RAS neutralizing antibody-CPP conjugate of the present invention may be used as separately administered compositions or in combination with other drugs, such as cytotoxic, cytostatic or immunotherapeutic drugs, and more particularly such as cyclosporine, methotrexate, adriamycin, cisplatinum or taxoids (placlitaxel, doxorubicin, etc.).

The invention further provides a method for the treatment or prevention of cancers or adenocarcinomas in a subject comprising administering to the subject, in an amount effective for said treatment or prevention, an anti-activated RAS neutralizing antibody-cell-penetrating peptide conjugate of the invention. The antibody-CPP conjugate used for treatment may be in the form of a composition, preferably a pharmaceutical composition, comprising said conjugate and a pharmaceutically acceptable carrier. Advantageously, the antibody is capable of selectively binding intracellular activated RAS protein *in vitro* and/or *in vivo* and consequently the activated RAS protein is functionally inactivated.

Thus, the present invention relates to a chimeric polypeptide **characterized in that** said polypeptide comprises a first domain and a second domain, wherein
- the first domain comprises an amino-acid sequence facilitating active transport across a biological membrane by the binding to an glycosaminoglycan, which is selected from the group consisting of a) (XBBBXXBX)n ; b) (XBBXBX)n ; c) (BBXmBBXp)n ; d) (XBBXXBX)n ; e) (BXmBB)n ; f) (BmXX)n or g) an antibody fragment, wherein each B is independently a basic amino acid preferably lysine or arginine; each X is independently a non-basic amino acid preferably hydrophobic amino acid; each m is independently an integer from zero to five; each n is independently an integer between one and ten; and each p is independently an integer between zero to five; and
- the second domain comprises an anti-activated RAS neutralizing antibody, fragment or derivative thereof.

The term "peptide(s)" refer to a polymer of amino acids of which the written convention is N, or amino, terminus is on the left and the C, or carboxyl, terminus is on the right. The 20 most common, natural L-amino acids are alternatively designated by three-letter or one-letter codes. Peptides, as used herein, are considered to include "peptide analogs", structural modifications containing one or more modifications to L-amino acid side-chains or to the alpha-amino acid backbone. An example of a backbone modified peptide analog is the N-methyl glycine "peptoid" (Zuckermann et al., J. Amer. Chem. Soc. 114:10646-47 (1992)).

The term "cell penetrating peptide(s)" (CPP(s)) is defined as a carrier peptide that is capable of crossing biological membrane or a physiological barrier. Cell penetrating peptides are also called cell-permeable peptides, protein-transduction domains (PTD) or membrane-translocation sequences (MTS). CPPs have the ability to translocate *in vitro* and/or *in vivo* the mammalian cell membranes and enter into cells, and directs a conjugated compound of interest, such as a drug or marker, to a desired cellular destination, *e.g*. into he cytoplasm (cytosol, endoplasmic reticulum, Golgi apparatus, etc.) or the nucleus. Accordingly, the CPP can direct or facilitate penetration of a compound of interest across a phospholipid, mitochondrial, endosomal or nuclear membrane. The CPP can also direct a compound of interest from outside the cell through the plasma membrane, and into the cytoplasm or to a desired location within the cell, *e.g*., the nucleus, the ribosome, the mitochondria, the endoplasmic reticulum, a lysosome, or a peroxisome. Alternatively or in addition, the CPP can direct a compound of interest across the blood-brain, trans-mucosal, hematoretinal, skin, gastrointestinal and/or pulmonary barriers.

Penetration across a biological membrane or a physiological barrier can be determined by various processes, for example by a cell penetration test having a first incubation step for the CPP conjugated to a marker in the presence of culture cells, followed by a fixating step, and then revelation of the presence of the marked peptide inside the cell. In another embodiment, the revelation step can be done with an incubation of the CPP in the presence of labeled antibodies and directed against the CPP, followed by detection in the cytoplasm or in immediate proximity of the cell nucleus, or even within it, of the immunologic reaction between the CPP's amino acid sequence and the labeled antibodies. Revelation can also be done by marking an amino acid sequence in the CPP and detecting the presence of the marking in the cell compartments. Cell penetration tests are well known to those skilled in the art. However, for example a cell penetration test was described in patent application No WO 97/02840.

Several proteins and their peptide derivatives have been found to possess cell internalization properties including but not limited to the Human Immunodeficency Virus type 1 (HIV-1) protein Tat (Ruben et al. J. Virol. 63, 1-8 (1989)), the herpes virus tegument protein VP22 (Elliott and O'Hare, Cell 88, 223-233 (1997)), the homeotic protein of *Drosophila melanogaster* Antennapedia (the CPP is called Penetratin) (Derossi et al., J. Biol. Chem. 271, 18188-18193 (1996)), the protegrin 1 (PG-1) anti-microbial peptide SynB (Kokryakov et al., FEBS Lett. 327, 231-236 (1993)) and the basic fibroblast growth factor (Jans, Faseb J. 8, 841-847 (1994)). A number of other proteins and their peptide derivatives have been found to possess similar cell internalization properties. The carrier peptides that have been derived from these proteins show little sequence homology with each other, but are all highly cationic and arginine or lysine rich. Indeed, synthetic poly-arginine peptides have been shown to be internalized with a high level of efficiency (Futaki et al., J. Mol. Recognit. 16, 260-264 (2003); Suzuki et al., J. Biol. Chem. (2001)).

CPP can be of any length. For example CPP is less than or equal to 500, 250, 150, 100, 50, 25, 10, 6 or 4 amino acids in length. For example CPP is greater than or equal to 4, 5, 6, 10, 25, 50, 100, 150 or 250 amino acids in length. The suitable length and design of the CPP will be easily determined by those skilled in the art. As general references on CPPs it can be cited: CELL PENETRATING PEPTIDES: PROCESSES AND APPLICATIONS, edited by Ulo Langel (2002); or Advanced Drug Delivery Reviews 57:489-660 (2005); or Dietz and Bähr, Moll Cell. Neurosci. 27:85-131 (2004).

In preferred embodiments the CPP is 4, 5, 6, 7, 5, 6, 7, 8, 9, 10, 11, 12, 13, 14, 15, 16, 17, 18, 19, 20, 21, 22, 23, 24 or 25 amino acids in length.

The cell penetrating peptides according to the invention can be, but not limited to, those described below or variants thereof. A "variant" that is at least about 50%, preferably at least about 70%, more preferably at least about 80%-85%, preferably at least about 90% and most preferably at least about 95%-99% identical thereto. For example, peptides can have substitutions at 1, 2, 3, 4 or more residues. The CPP can be used in their natural form (such as described above) or polymer form (dimer, trimer, etc.).

**Table 1: Selection of well-known cell penetrating peptide used for cargo delivery**

| SEQ ID NO: | Cell Penetrating Peptides | Amino acid sequences (Nter to Cter) in one letter code |
|---|---|---|
| 1 | Buforin II | TRSSRAGLQFPVGRVHRLLRK |
| 2 | DPV3 | RKICRRRESRKICRRRES |
| 3 | DPV6 | GRPRESGKICRICRKRLKP |
| 4 | DPV7 | GKRKKKGKLGKKRDP |
| 5 | DPV7b | GKRKKKGKLGKKRPRSR |
| 6 | DPV3/10 | RKICRRRESRRARRSPRHL |
| 7 | DPV10/6 | SRRARRSPRESGKKRKRKR |
| 8 | DPV1047 | VKRGLKLRHVRPRVTRMDV |
| 9 | DPV1048 | VKRGLKLRHVRPRVTRDV |
| 10 | DPV10 | SRRARRSPRHLGSG |
| 11 | DPV15 | LRRERQSRLRRERQSR |
| 12 | DPV15b | GAYDLRRRERQSRLRRRERQSR |
| 13 | GALA | WEAALAEALAEALAEHLAEALAEALEALAA |
| | Haptotactic peptides | |
| 14 | Cβ | KGSWYSMRKMSMKIRPFFPQQ |
| 15 | preCγ | KTRYYSMKKTTMKIIPFNRL |
| 16 | CaE | RGADYSLRAVRMKIRPLVTQ |
| 17 | hCT(9-32) | LGTYTQDFNKFHTFPQTAIGVGAP |
| 18 | HN-1 | TSPLNIHNGQKL |
| 19 | Influenza virus nucleoprotein (NLS) | NSAAFEDLRVLS |
| 20 | KALA | WEAKLAKALAKALAKHLAKALAKALKACEA |
| 21 | Ku70 | VPMLKPMLKE |
| 22 | MAP | KLALKLALKALKAALKLA |
| 23 | MPG | GALFLGFLGAAGSTMGAWSQPKKICRKV |
| 24 | MPM (IP/K-FGF) | AAVALLPAVLLALLAP |
| 25 | N50 (NLS of NF-κB P50) | VQRKRQKLM |
| 26 | Pep-1 | KETWWETWWTEWSQPKKKRKV |
| 27 | Pep-7 | SDLWEMMMVSLACQY |
| 28 | Penetratin | RQIKIWFQNRRMKWKK |
| 29 | Short Penetratin | RRMKWKK |
| 30 | Poly Arginine - R₇ | RRRRRRR |
| 31 | Poly Arginine - R₉ | RRRRRRRRR |
| 32 | pISL | RVIRVWFQNKRCKDKK |
| 33 | Prion mouse PrPc₁₋₂₈ | MANLGYWLLALFVTMWTDVGLCKKRPKP |
| 34 | pVEC | LLIILRRRIRKQAHAHSK |
| 35 | SAP | VRLPPPVRLPPPVRLPPP |
| 36 | SV-40 (NLS) | PKKKRKV |
| 37 | SynB1 | RGGRLSYSRRRFSTSTGR |
| 38 | SynB3 | RRLSYSRRRF |
| 39 | SynB4 | AWSFRVSYRGISYRRSR |
| 40 | Tat₄₇₋₆₀ | YGRKKRRQRRRPPQ |
| 41 | Tat₄₇₋₅₇ | YGRKKRRQRRR |
| 42 | Tat₄₉₋₅₇ | RKKRRQRRR |
| 43 | Transportan | GWTLNSAGYLLGKINLKALAALAKKIL |
| 44 | Transportan 10 | AGYLLGKINLKALAALAKKIL |
| 45 | Transportan derivative: | GWTLNSAGYLLG |
| 46 | Transportan derivative | INLKALAALAKKIL |
| 47 | VP22 | DAATATRGRSAASRPTERPRAPARSASRPRRPVD |
| 48 | VT5 | DPKGDPKGVTVTVTVTVTGKGDPKPD |

If necessary, several well known chemical strategies can be used by one skilled in the art for transforming a CPP into a drug candidate with increased stability *in vivo,* bioavailability and/or biological activity; such as:
- N- and C-terminus modifications to prevent exopeptidase degradation: C-terminal amidation or N-terminal acetylation increases peptide lipophilicity,
- cyclization by forming a disulfide bridge,
- alkylation of amide nitrogen to prevent endopeptidase degradation,
- introduction of non-natural amino acids to modify the recognition site of the endopeptidase (2-methylalanine, alpha-dialkylated glycine, oligocarbamate, oligourea, guanidino or amidino backbones...),
- incorporation of non-genetically encoded amino acids (methylation, halogenation or chlorination of glycine or phenylalanine) into the CPP amino acid sequence,
- replacement of some or even all the L-amino acids with their corresponding D-amino acid or beta-amino acid analogues. Such peptides may be synthesized as "inverso" or "retro-inverso" forms, that is, by replacing L-amino acids of the sequence with D-amino acids, or by reversing the sequence of the amino acids and replacing the L-amino acids with D-amino acids. Structurally, the retro-inverse peptide is much more similar to the original peptide than the simple D-analogue. D-peptides are substantially more resistant to peptidases, and therefore are more stable in serum and tissues compared to their L-peptide counterparts. In a preferred embodiment CPPs containing L-amino acids are capped with a single D-amino acid to inhibit exopeptidase destruction,
- synthesis of CPP-derived oligocarbamate; the oligocarbamate backbone consists of a chiral ethylene backbone linked through relatively rigid carbamate groups (Cho et al., Science 261:1303-1305 (1993)).

In another embodiment, the CPP contains contiguous or non- contiguous basic amino acid or amino acid analog, particularly guanidyl or amidinyl moieties. The terms "guanidyl" and "guanidine" are used interchangeably to refer to a moiety having the formula -HN=C(NH₂)NH (unprotonated form). As an example, arginine contains a guanidyl (guanidino) moiety, and is also referred to as 2-amino-5-guanidinovaleric acid or a-amino-6-guanidinovaleric acid. The terms "amidinyl" and "amidino" are used interchangeably and refer to a moiety having the formula -C(=NH)(NH2). A "basic amino acid or amino acid analog" has a side chain pKa of greater than 10. Preferred highly basic amino acids are histidine, arginine and/or lysine.

In a preferred embodiment, the CPP according to the invention comprises at least 1, 2, 3, 4, 5, 6, 7, 8, 9, 10 basic amino acid or amino acid analog, particularly lysine or arginine.

According to a more preferred embodiment, the CPP is further characterized by its ability to react with or bind to glycosaminoglycans (GAGs) (long unbranched molecules containing a repeating disaccharide unit) or specifically hyaluronic acid, heparin, heparan sulfate, dermatan sulfate, keratin sulfate or chondroitin sulfate and their derivatives. "Heparin, heparan sulfate or chondroitin sulfate derivatives" or "glycosaminoglycans" are understood to mean any product or sub-product as defined in the publications cited in references (Cardin and Weintraub, Arteriosclerosis 9: 21 (1989); Merton et al., Annu. Rev. Cell Biol. 8: 365 (1992); David, FASEB J. 7: 1023 (1993)).

The capacity of the CPPs to react with / bind to glycosaminoglycans (GAGs) can be determined by direct or indirect glycosaminoglycan-binding assays known in the art, such as the affinity co-electrophoresis (ACE) assay for peptide glycosaminoglycan binding described in the PCT patent application WO 00/45831. Several other methods well known in the art are available for analyzing GAG-peptides interactions, for example the method described in the PCT patent application WO 01/64738 or by Weisgraber and Rall (J. Biol. Chem., 262(33):11097-103) (specific example with the apolipoprotein B-100); or by a modified ELISA test: 96-well plates are coated with specific GAG (chondroitin sulfate A, B and C, heparin, heparan sulfate, hyaluronic acid, keratan sulfate, syndecan), peptide conjugated to a marker is then added for a defined time; after extensive washing, peptide binding is determined using specific analysis related to the marker.

CPPs capable of reacting *in vitro* and/or *in vivo* with glycosaminoglycans were described in the patent applications No WO 01/64738 and No WO 05/016960 and by De Coupade et al. (Biochem J. 390:407-18 (2005)). These peptides are amino acid sequences originating from human heparin binding proteins and/or anti-DNA antibodies selected from the group comprising: the lipoproteins such as human apolipoprotein B or E (Cardin et al., Biochem. Biosphys. Res. Com. 154: 741 (1988)), the agrine (Campanelli et al., Development 122: 1663-1672 (1996)), the insulin growth factor binding protein (Fowlkes et al., Endocrinol. 138: 2280-2285 (1997)), the human platelet-derived growth factor (Maher et al., Mol. Cell. Biol. 9: 2251-2253 (1989)), the human extracellular superoxide dismutase (EC-SOD) (Inoue et al., FEBS 269: 89-92 (1990)), the human heparin-binding epidermal growth factor-like growth factor (HB-EGF) (Arkonac et al., J. Biol. Chem. 273: 4400-4405 (1998)), the acid fibroblast growth factor (aFGF) (Fromm et al., Arch. Biochem. Bioph. 343: 92 (1997)), the basic fibroblast growth factor (bFGF) (Yayon et al., Cell 64: 841-848 (1991)), the human intestinal mucin 2 sequence (Xu et al., Glyconjug J. 13: 81-90 (1996)), the human gamma interferon (Lortat-Jacob & Grimaud, FEBS 280: 152-154 (1991)), the subunit p40 of human interleukin 12 (Hasan et al., J. Immunol. 162: 1064-1070 (1999)), the factor 1-alpha derived from stromal cells (Amara et al., J. Biol. Chem. 272: 200-204 (1999)), the human neutrophil derived "heparin binding protein" (CAP 37/azurocidin) (Pohl et al., FEBS 272: 200-204 (1990)), an immunoglobulin molecule such as CDR2 and/or CDR3 regions of the anti-DNA monoclonal murine antibody F4.1 (Avrameas et al., Proc. Natl. Acad. Sci. 95: 5601 (1998)), the hyper variable CDR3 region of human anti-DNA monoclonal antibody RTT79 (Stevenson et al., J. Autoimmunity 6: 809 (1993)), the hyper variable area CDR2 and/or CDR3 of the human anti-DNA monoclonal antibody NE-1 (Hirabayashi et al., Scand. J. Immunol. 37: 533 (1993)), the hypervariable area CDR3 of the human anti-DNA monoclonal antibody RT72 (Kalsi et al, Lupus 4: 375 (1995)).

According to a more preferred embodiment, the CPP comprises an amino-acid sequence selected from the group consisting of a) (XBBBXXBX)n ; b) (XBBXBX)n ;c) (BBXmBBXp)n ; d) (XBBXXBX)n; e) (BXBB)n and f) (an antibody fragment), wherein each B is independently a basic amino acid preferably lysine or arginine; each X is independently a non-basic amino acid preferably hydrophobic amino acid, such as alanine, isoleucine, leucine, methionine, phenylalanine, tryptophan, valine, tyrosine or citrulline; each m is independently an integer from zero to five; each n is independently an integer between one and ten; and each p is independently an integer between zero to five. In certain embodiments n may be 2 or 3 and X may be a hydrophobic amino acid. An antibody fragment is meant to include a less than full-length immunoglobulin polypeptide, *e.g*., a heavy chain, light chain, Fab, Fab'2, Fv or Fc. The antibody can be for example human or murine. Preferably the antibody is an anti-DNA antibody. Preferably, the antibody fragment contains all or part of the CDR2 region of an antibody, particularly at least a portion of at least 6 or 10 amino acids in length of a CDR2 region of an anti-DNA antibody. Alternatively, the antibody contains all or part of the CDR3 region of an antibody, particularly at least a portion of at least 6 or 12 amino acids in length of a CDR3 region of an anti-DNA antibody. More specifically, the antibody fragment contains at least one CDR3 region of an anti-DNA human antibody, such as RTT79, NE-1 and RT72. Such antibody fragments have been described in PCT patent application n° WO 99/07414.

Preferably, the CPP according to the invention is further **characterized in that** it is originating from human proteins (*i.e*., proteins naturally expressed by human cells). Thus, the characteristic of CPPs derived from human proteins compared to the CPPs derived from non-human proteins, is of primary interest in the planned use of these CPPs, since their immunogenicity is avoided or lowered. In addition, De Coupade et al., (Biochem J. 390:407-18 (2005)) have shown that human-derived peptides have low *in vivo* toxicity profiles consistent with their use as therapeutic delivery systems, unlike existing carrier peptides such as Tat peptides (Trehin and Merkle, Eur. J. Pharm. Biopharm. 58, 209-223 (2004)).

Among the CPPs described above, preferred are those capable of specifically penetrating into the cytoplasm. Penetration of a CPP into the cytoplasm can be determined by various processes *in vitro* well known by one skilled in the art: for example by incubating the CPP with cells; then, the cells are incubated in the presence of specific anti-CPP labeled antibodies and specific anti-cytoplasm protein labeled antibodies, followed by detection in the cytoplasm of the immunologic reaction between the CPP and the labeled antibodies. Another method is to conjugate the CPP to colloidal gold and incubate the conjugate with cells. The cells are then treated as usual for the electron microscope to visualise the intracellular localization.

Accordingly, preferred CPPs, derived from human heparin binding protein and capable of specifically penetrate into the cytoplasm of a target cell are selected from the group comprising:
- DPV6 (SEQ ID NO: 3): CPP reacting with heparin and derived from the amino acid sequence of the C-terminal part of chain A of the human platelet-derived growth factor (Maher et al., Mol. Cell. Biol. 9: 2251-2253 (1989)). It comprises an amino acid sequence of formula c) (BBXmBBXp)n wherein m = 0, p = 0 and n = 1;
- DPV7 (SEQ ID NO: 4) and DPV7b (SEQ ID NO: 5): CPPs reacting with heparin and derived from the C-terminal part of the sequence of the human heparin-binding epidermal growth factor-like growth factor (HB-EGF) (Arkonac et al., J. Biol. Chem. 273: 4400-4405 (1998)). Both CPP comprise an amino acid sequence of formula c) (BBXmBBXp)n wherein m = 0, p = 0 and n = 1;
- DPV10 (SEQ ID NO: 10): CPP reacting with heparin and corresponding to the C-terminal part of the human intestinal mucin 2 sequence (Xu et al., Glyconjug J. 13: 81-90 (1996)). It comprises an amino acid sequence of formula e) (BXBB)n wherein n = 1;
- DPV3/10 (SEQ ID NO: 6): CPP reacting with heparin and derived from the C-terminal part of the sequence of human extracellular superoxide dismutase (EC-SOD) (see above) and from C-terminal part of the human intestinal mucin 2 sequence (see above). It comprises an amino acid sequence of formula c) (BBXmBBXp)n wherein m = 1, p = 2 and n = 1;
- DPV10/6 (SEQ ID NO: 7): CPP reacting with heparin and derived from the C-terminal part of the human intestinal mucin 2 sequence (see above) and from the C-terminal part of chain A of the platelet-derived growth factor (see above). It comprises an amino acid sequence of formula c) (BBXmBBXp)n wherein m = 1, p = 2 and n = 1;
- DPV1047 (SEQ ID NO: 8): CPP reacting with heparin derived from the amino acid sequence (3358-3372) of the human lipoprotein B (Cardin et al., Biochem. Biosphys. Res. Com. 154: 741 (1988)) and from the sequence of the peptide corresponding to the hypervariable area CDR3 of the human anti-DNA monoclonal antibody NE-1 (Hirabayashi et al., Scand. J. Immunol. 37: 533 (1993)). It comprises an amino acid sequence of formula d) (XBBXXBX)n wherein n = 1, and an antibody fragment or an amino acid sequence of formula b) (XBBXBX)n wherein n = 1;
- DPV15b (SEQ ID NO: 11): CPP reacting with heparin and containing part of the sequence of the "heparin binding protein" (CAP 37). It comprises an amino acid sequence of formula d) (XBBXXBX) repeated twice.

A more preferred CPP is DPV3 (SEQ ID NO: 2): CPP reacting with heparin and dimer of a peptide derived from the C-terminal part of the sequence of human extracellular superoxide dismutase (EC-SOD) (Inoue et al., FEBS 269: 89-92 (1990)). It comprises an amino acid sequence of formula c) (BBXmBBXp)n wherein m = 0, p = 0 and n = 1. DPV3 contains the amino acid sequence BBBBBBXXBBBBBBXX.

A "p21-RAS protein" or "RAS protein" is defined as a protein encoded by a *ras* gene. The *ras* gene family is found in a wide variety of nucleated mammalian cells and participates in normal cellular function. It consists of three functional genes, H-*ras,* N-*ras,* and K-*ras.* The *ras* genes encode 21kDa proteins (designated p21), which are associated with the inner plasma membrane (H-RAS, N-RAS, and the alternatively spliced K-RAS A and K-RAS B). Whereas H-RAS, N-RAS, and K-RAS B are ubiquitously expressed, K-RAS A is induced during differentiation of pluripotent embryonal stem cells *in vitro* (Pells et al., Oncogene 15, 1781-1786 (1997)).

An "activated RAS protein" or "activated RAS" is defined as the form of a RAS protein which is capable of inducing cells to lose their normal growth control. As used herein, the term "activated RAS protein" is synonymous with "oncogenic RAS protein". It has been shown that activated RAS proteins frequently contain point mutations (an amino acid substitution) at position 12, 13 or 61 (Reddy et al., Nature 300, 149-152 (1982) ; Tabin et al., Nature 300, 143-149 (1982) and Bos, Cancer Res. 49, 4682-4689 (1989)).

An "anti-activated RAS neutralizing antibody, fragment or derivative thereof" is defined as a neutralizing antibody, fragment or derivative thereof, which specifically binds to an epitope on the activated RAS proteins, but does not bind an epitope of normal, non-activated RAS protein. The term "specific binding to an epitope on activated RAS protein" means that within a mixture of reagents comprising activated RAS in addition to other alternative antigens, only activated RAS is bound. Preferably, the specific binding of an antibody according to the present invention to activated RAS is of high affinity, *e.g*., between about 10⁻¹¹ and about 10⁻¹⁰ molar. Affinity measurements may be made using methods known to those skilled in the art, including using the method of equilibrium dialysis.

A "neutralizing antibody, fragment or derivative thereof" means an antibody, fragment or derivative thereof, whose binding to an activated RAS protein results in inhibition of the biological activity (*e.g*. function) of said activated RAS protein. This inhibition of the biological activity can be assessed by measuring one or more indicators of the activated RAS protein biological activity, such as the nucleotide exchange reaction between p21-bound and exogenous guanine nucleotides, entry in S-phase DNA synthesis and cell replication (Hattori et al., Mol Cell Biol 7, 1999-2002 (1987)). The term "inhibition" means that the cell transforming ability of activated RAS is inhibited as compared with a suitable control in which control cells are not treated with an antibody of the present invention. Advantageously, the cell transforming ability of activated RAS is inhibited by 20% as compared with a suitable control. More advantageously, it is inhibited by 30%, 40%, 50%, 60%, 70%, 80% or 90%. In a most preferred embodiment of this aspect of the invention, the cell transforming ability of activated RAS is inhibited by 100% as compared with a suitable control. As referred to herein the term the "transforming ability" (of activated RAS) refers to the ability of activated RAS to induce cells to lose their normal growth control. For example "transformed cells" undergo endless replication and exhibit loss of contact inhibition.

The term "antibody" according to the present invention, refers to immunoglobulin molecules and immunologically active portions of immunoglobulin molecules, *i.e*., molecules that contain an antigen binding site that immunospecifically binds an activated RAS protein. The immunoglobulin molecules of the invention can be of any type (*e.g*., IgG, IgE, IgM, IgD, IgA and IgY), class (*e.g*. IgG1, IgG2, IgG3, IgG4, IgA1 and IgA2) or subclass of immunoglobulin molecule. Preferably, the antibodies are human, murine (*e.g*. mouse and rat), donkey, sheep, rabbit, goat, guinea pig, camelid, horse, or chicken. immunoglobulin molecules of the invention can be polyclonal or monoclonal, preferably monoclonal antibodies.

As used herein, "human" antibodies include antibodies having the amino acid sequence of a human immunoglobulin and include antibodies isolated from human immunoglobulin libraries, from human B cells, or from animals transgenic for one or more human immunoglobulin, as described for example in U.S. Patent No. 5,939,598.

The term "antibody fragment" according to the present invention, refers to antibodies that are anti-activated RAS neutralizing antibody fragments and include, but are not limited to, Fab, Fab' and F(ab')2, Fd, single-chain Fvs (scFv) , single-chain antibodies, disulfide- linked Fvs (sdFv) and fragments comprising either a VL or VH domain. Anti-activated RAS neutralizing antibody fragments, including single-chain antibodies, may comprise the variable region(s) alone or in combination with the entirety or a portion of the following: hinge region, CH1, CH2, CH3 and CL domains. Also included in the invention are anti-activated RAS neutralizing antibody fragments comprising any combination of variable region(s) with a hinge region, CH1, CH2, CH3 and CL domains.

The term "derivatives" according to the present invention, refers to antibodies or fragments thereof (activated RAS protein binding fragments of any of the above) having an identity of more than 60% identity with an antibody or fragments thereof as defined above, preferably more than 70% identity, as an example more than 80% identity or more than 90% identity, and most preferably more than 95% identity with an antibody or fragments thereof as defined above.

Preferably, said derivatives correspond to humanized antibodies or fragments thereof.

The antibodies of the present invention may be generated by any suitable method known in the art. Polyclonal antibodies to an activated RAS protein can be produced by various procedures well known in the art. For example, an activated RAS protein can be administered to various host animals including, but not limited to, rabbits, mice, rats, etc. to induce the production of sera containing polyclonal antibodies specific for the protein. Various adjuvants may be used to increase the immunological response, depending on the host species, and include but are not limited to, Freund's (complete and incomplete), mineral gels such as aluminum hydroxide, surface active substances such as lysolecithin, pluronic polyols5 polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed., 1988); Hammerling, et al., in: Monoclonal Antibodies and T-Cell Hybridomas 563- 681 (Elsevier, N.Y., 1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Methods for producing and screening for specific antibodies using hybridoma technology are routine and well known in the art.

The methods for making a "humanized antibody" are well-known in the art. This is a technique of reconstructing the antigen binding site of a non-human antibody (*e.g*. a rodent antibody) on a human antibody (Jones, P. T. et al., Nature 321, 5225-525 (1986); Verhoeyen, M. et al., Science 239, 1534-1536 (1988); Riechmann, L. et al., Nature 332,323-327 (1988)). In general, the humanized antibody or fragment thereof comprises substantially at least one, and more preferably two, variable domains (Fab, Fab', F(ab')2, Fabc, Fv) in which all or substantially all of the complementarity determining regions (CDR) correspond to those of a non-human immunoglobulin, and all or substantially all of the framework regions (FR) are those of a human immunoglobulin consensus sequence. The humanized antibody optimally also comprises at least a portion of an immunoglobulin constant region (Fc), typically that of a human immunoglobulin. Ordinarily, the antibody contains both the light chain as well as at least the variable domain of a heavy chain. The antibody also may include the CH1, hinge, CH2, CH3, and CH4 regions of the heavy chain. The humanized antibody may be selected from any class of immunoglobulins, including IgM, IgG, IgD, IgA and IgE, and any isotype, including IgG1, IgG2, IgG3 and IgG4. Usually the constant domain is a complement fixing constant domain where it is desired that the humanized antibody exhibit cytotoxic activity, and the class is typically IgG and preferably IgG1. Where such cytotoxic activity is not desirable, the constant domain may be of the IgG2 class. The humanized antibody may comprise sequences from more than one class or isotype, and selecting particular constant domains to optimize desired effector functions is within the ordinary skill in the art. The FR and CDR regions of the humanized antibody need not correspond precisely to the parental sequences, *e.g.*, the import CDR or the consensus FR may be mutagenized by substitution, insertion or deletion of one or more residues so that the CDR or FR residue at that site does not correspond to either the consensus or the import antibody. Such mutations, however, will not be extensive and will not dramatically affect binding of the antibody to the binding target. The expression "humanized antibody" also includes hybrid and recombinant antibodies and polypeptides produced by splicing a variable region or one or more CDRs of an anti-AF-20 antibody with any heterologous protein(s), regardless of species of origin, type of protein, immunoglobulin class or subclass designation, so long as the hybrid and recombinant antibodies and polypeptides exhibit the desired biological activity. The expression "humanized antibody" further includes antibodies and polypeptides rendered non-immunogenic, or having reduced immunogenicity relative to the native antibody, to a human by the method of determining at least part of the amino acid sequence of the antibody or polypeptide (preferably that part of non-human origin such as a VH or VK region of a non-human antibody), identifying in the amino acid sequence one or more potential epitopes for human T-cells, and modifying the amino acid sequence(s) of the one or more epitopes to eliminate at least one of the T-cell epitopes identified in order to eliminate or reduce the immunogenicity of the protein or portions thereof when exposed to the human immune system. In the creation of a humanized antibody, preferably about 75%, more preferably about 90%, and most preferably greater than about 95% of the humanized antibody residues will correspond to those of the parental FR and CDR sequences.

Antibody fragments which recognize specific epitopes on an activated RAS protein may be generated by known techniques. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH 1 domain of the heavy chain.

In a specific embodiment, the antibody is the rat neutralizing monoclonal antibody Y13259 or a derivative thereof (Furth et al., J. Virol. 43, 294-304 (1982); Kung et al., Exp. Cell Res. 162, 363-371 (1986); Hattori et al., Mol. Cell Biol. 7, 1999-2002(1987); Hamer et al., Hybridoma 9, 573-587 (1990). The term "derivative" is intended to encompass any form of the Y13259 antibody as defined above, wherein one or more of the amino acids within the polypeptide chain has been replaced with an alternative amino acid and/or wherein one or more of the amino acids has been deleted or wherein one or more additional amino acids has been added to the polypeptide chain or amino acid sequences of Y13259 antibody, which still has at least one function of the native Y13259 antibody, *i.e*. which is still an anti-activated RAS neutralizing antibody. A Y13259 derivative exhibits at least about 50%, preferably at least about 70%, more preferably at least about 80%-85%, preferably at least about 90% and most preferably at least about 95%-99% amino acid sequence homology over the defined native Y13259 antibody. Many suitable computer programs for calculating the "homology" between two amino acid sequences are generally known in the art, such as BLAST program (http://www.ncbi.nim.nih.gov/BLAST/) choosing the scoring matrix BLOSUM62.

In a specific embodiment, the rat neutralizing monoclonal antibody Y13259 is produced by the hybridoma clone named Y13259 that originated from ATCC, number CRL-1742

The CPP and the anti-activated RAS neutralizing antibody can be conjugated (*i.e*., coupled by chemical coupling in any suitable manner known in the art. Many known chemical cross-linking methods are non-specific, *i. e*., they do not direct the point of coupling to any particular site on CPP or the suitable anti-activated RAS neutralizing antibody. As a result, use of non-specific cross-linking reagents may attack functional sites or sterically block active sites, rendering the conjugated proteins biologically inactive. The anti-activated RAS neutralizing antibody can be coupled directly to the CPP either on one of those terminal ends (N or C terminus) or on a side chain or one of the amino acids. The anti-activated RAS neutralizing antibody can also be coupled indirectly by a connecting arm either to one of the terminal ends of the peptides or to a side chain of one of the amino acids.

One way to increasing coupling specificity is to directly chemical coupling to a functional group found only once or a few times in one or both of the polypeptides to be cross-linked. For example, in many proteins, cysteine, which is the only protein amino acid containing a thiol group, occurs only a few times. Also, for example, if a polypeptide contains no lysine residues, a cross-linking reagent specific for primary amines will be selective for the amino terminus of that polypeptide. Successful utilization of this approach to increase coupling specificity requires that the polypeptide have the suitably rare and reactive residues in areas of the molecule that may be altered without loss of the molecule's biological activity.

Cysteine residues may be replaced when they occur in parts of a polypeptide sequence where their participation in a cross-linking reaction would otherwise likely interfere with biological activity. When a cysteine residue is replaced, it is typically desirable to minimize resulting changes in polypeptide folding. Changes in polypeptide folding are minimized when the replacement is chemically and sterically similar to cysteine. For these reasons, serine is preferred as a replacement for cysteine. As demonstrated in the examples below, a cysteine residue may be introduced into a polypeptide's amino acid sequence for cross-linking purposes. When a cysteine residue is introduced, introduction at or near the amino or carboxy terminus is preferred. Conventional methods are available for such amino acid sequence modifications, whether the polypeptide of interest is produced by chemical synthesis or expression of recombinant DNA.

Coupling of the two constituents can be accomplished via a cross-linking reagent. There are several intermolecular cross-linking reagents which can be utilized, see for example, Means and Feeney, CHEMICAL MODIFICATION OF PROTEINS, Holden-Day, 1974, pp. 39-43. Among these reagents are, for example, *N*-succinimidyl 3- (2-pyridyldithio) propionate (SPDP) or N, N'- (1,3-phenylene) bismaleimide (both of which are highly specific for sulfhydryl groups and form irreversible linkages); N, N'-ethylene-bis- (iodoacetamide) or other such reagent having 6 to 11 carbon methylene bridges (which relatively specific for sulfhydryl groups); and1, 5-difluoro-2,4-dinitrobenzene (which forms irreversible linkages with amino and tyrosine groups). Other cross-linking reagents useful for this purpose include: p, p'-difluoro-N, N'-dinitrodiphenylsulfone (which forms irreversible cross-linkages with amino and phenolic groups); dimethyl adipimidate (which is specific for amino groups); phenol-1, 4-disulfonylchloride (which reacts principally with amino groups); hexamethylenediisocyanate or diisothiocyanate, or azophenyl-p-diisocyanate (which reacts principally with amino groups); glutaraldehyde (which reacts with several different side chains) and disdiazobenzidine (which reacts primarily with tyrosine and histidine).

Cross-linking reagents may be homobifunctional, *i. e*., having two functional groups (*i.e*. reactive groups) that undergo the same reaction. An example of homobifunctional cross-linking reagent is bismaleimidohexane ("BMH"). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible cross-linking of polypeptides that contain cysteine residues.

Cross-linking reagents may also be heterobifunctional. Heterobifunctional cross-linking reagents have two different functional groups, for example an amine-reactive group and a thiol-reactive group, that will cross-link two proteins having free amines and thiols, respectively. Preferred heterobifunctional cross-linking reagents are succinimidyl 4- (N- maleimidomethyl) cyclohexane-1-carboxylate ("SMCC"), N-maleimidobenzoyl-N-hydroxysuccinimide ester ("MBS"), and succinimide 4- (p-maleimidophenyl) butyrate ("SMPB"), an extended chain analog of MBS. The succinimidyl group of these cross-linking reagents reacts with a primary amine forming an amide bond, and the thiol-reactive maleimide forms a covalent thioether bond with the thiol of a cysteine residue.

Cross-linking reagents often have low solubility in water. A hydrophilic moiety, such as a sulfonate group, may be added to the cross-linking reagent to improve its water solubility. Sulfo-MBS and sulfo-SMCC are examples of cross-linking reagents modified for water solubility.

Many cross-linking reagents yield a conjugate that is essentially non-cleavable under cellular conditions. However, some cross-linking reagents contain a covalent bond, such as a disulfide, that is cleavable under cellular conditions. For example, Traut's reagent, dithiobis(succinimidylpropionate) ("DSP"), and N-succinimidyl 3- (2-pyridyldithio) propionate ("SPDP") are well-known cleavable cross-linking reagents. Another example is the hydrazine derivatives such as the 4-(-4-*N-*Maleimidophenyl)butyric acid hyrazide (MPBH), 4-(*N*-Maleimidomethyl)cyclohexane-1-carboxyl-hydrazide (M₂C₂H), or the 3-(2-Pyridyldithio)propionyl hydrazide (PDPH). The use of a cleavable cross-linking reagent permits the lysosomal enzyme to separate from the CPP after delivery into the target cell. Direct disulfide linkage may also be useful.

Numerous cross-linking reagents, including the ones discussed above, are commercially available. Detailed instructions for their use are readily available from the commercial suppliers. A general reference on protein cross-linking and conjugate preparation is: Wong, CHEMISTRY OF PROTEIN CONJUGATION AND CROSS-LINKING, CRC Press (1991).

Chemical cross-linking may include the use of spacer arms. Spacer arms provide intramolecular flexibility or adjust intramolecular distances between conjugated domains and thereby may help preserve biological activity. A spacer arm may be in the form of a polypeptide domain that includes spacer amino acids, *e. g*. proline. Alternatively, a spacer arm may be part of the cross-linking reagent, such as in "long-chain SPDP" (Pierce Chem. Co.,Rockford, IL., cat. No. 21651 H).

In one embodiment of the invention, the CPP is coupled to the anti-activated RAS neutralizing antibody by at least one molecule (called an "anchoring molecule") that has a strong natural affinity for the anti-activated RAS neutralizing antibody. The natural affinity of the anchoring molecule for the anti-activated RAS neutralizing antibody allows the CPP to interact non-covalently with the anti-activated RAS neutralizing antibody, and hence to carry it along in intracellular travel. Another especially interesting advantage of this type of coupling consists of the fact that, due to the natural affinity of the anchoring molecule for the anti-activated RAS neutralizing antibody, these two elements are coupled in a totally natural way, with no chemical or biochemical interaction.

Alternatively, the chimeric polypeptide can be produced by genetic engineering as a fusion polypeptide that includes the CPP and the suitable anti-activated RAS neutralizing antibody sequence which can conveniently be expressed in known suitable host cells. Fusion polypeptides, as described herein, can be formed and used in ways analogous to or readily adaptable from standard recombinant DNA techniques. Accordingly, the present invention provides nucleic acid molecules and expression vectors comprising a nucleic acid sequence encoding an anti-activated RAS neutralizing antibody and a CPP of the invention. There are an abundance of expression vectors available and one skilled in the art could easily select an appropriate vector. In addition, standard laboratory manuals on genetic engineering provide recombinant DNA methods and methods for making and using expression vectors.

If desired, one or more amino acids can additionally be inserted between the first peptide domain comprising the CPP and the second polypeptide domain comprising the suitable anti-activated RAS neutralizing antibody. In some embodiments, the first or second domain includes a sequence that facilitates association of the CPP with the anti-activated RAS neutralizing antibody.

The invention also provides a method of treating or preventing a cancer by administering to a subject in which such treatment or prevention is desired a composition comprising an anti-activated RAS neutralizing antibody-CPP conjugate of the invention in an amount sufficient to treat or prevent the disease in the subject. In a specific embodiment, the cancer is a carcinoma comprising but not limited to an adenocarcinoma of the pancreas, the colon or the lung. Any composition can comprise an effective amount of a chimeric polypeptide of the invention from 0.1% to 99%, preferably 1% to 70%, of the composition. As example, the dosages of the chimeric polypeptide of the invention, when they are used for the effects indicated, will be between around 0.05 and 1,000 mg, and preferably 0.5, 1.0, 2.5, 5.0, 10.0, 15.0, 25.0, 50.0, 100.0, 250.0, 500.0 mg per composition. Efficaciousness of treatment is determined in association with any known method for diagnosing or treating cancers. The subject can be any mammal, *e. g*., a human, a primate, mouse, rat, dog, cat, cow, horse, pig.

The anti-activated RAS neutralizing antibody-CPP conjugates or nucleic acid molecules encoding these conjugates (also referred to herein as "therapeutics" or "active compounds") of the invention, and derivatives, fragments, analogs and homologs thereof, can be incorporated into pharmaceutical compositions suitable for administration; such compositions comprising a pharmaceutically acceptable carrier. In another embodiment the invention relates to the treatment of cancer with associations of at least one anti-activated RAS neutralizing antibody-CPP conjugate according to the invention and at least one other anti-tumor agents like the anthracyclins (such as doxorubicin) and the vinca alkaloids or an anti-tumor agents that have anti-inflammatory effects like the methotrexate, and the use of such associations for an improved treatment.

As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e*. *g*., intravenous, intradermal, subcutaneous, , transdermal (*i. e*., topical), and transmucosal, or oral (*e*. *g*., inhalation), rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic. Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL (BASF, Parsippany, N. J.), glucose or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (*e. g*., glycerol, propylene glycol, and liquid polyethylene glycol, and the like), and suitable mixtures thereof.

The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. For administration by inhalation, the compounds are delivered in the form of an aerosol spray from pressured container or dispenser which contains a suitable propellant, *e. g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compositions can also be prepared in the form of suppositories (*e. g*., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

In some embodiments, oral or parenteral compositions are formulated in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Sustained-release preparations can be prepared, if desired. Suitable examples of sustained-release preparations include semi-permeable matrices of solid hydrophobic polymers containing the active compound, which matrices are in the form of shaped articles, *e. g*., films, or microcapsules. Examples of sustained-release matrices include polyesters, hydrogels (for example, poly (2-hydroxyethyl-methacrylate), or poly (vinylalcohol)), polylactides (U. S. Pat. No. 3,773, 919), copolymers of L-glutamic acid and y ethyl-L- glutamate, non-degradable ethylene-vinyl acetate, degradable lactic acid-glycolic acid copolymers such as the LUPRON DEPOT (injectable microspheres composed of lactic acid-glycolic acid copolymer and leuprolide acetate), and poly-D- (-)-3-hydroxybutyric acid.

As examples, the oral dosages of the chimeric polypeptides of the invention, when they are used for the effects indicated, will be between around 0.05 and 1,000 mg/day by the oral route and, preferably come in the form of tablets containing 0.5, 1.0, 2.5, 5.0, 10.0, 15.0, 25.0, 50.0, 100.0, 250.0, 500.0 and 1,000.0 mg of active ingredient. The effective plasma levels of the vectors or transporters loaded with at least one substance of interest will range from 0.002 mg to 50 mg per kilogram of body weight and per day.

The chimeric polypeptides or nucleic acid encoding the chimeric polypeptides of the invention may be administered in the form of single daily doses, or the total daily dose may be administered in two, three or four doses per day.

The pharmaceutical compositions can be included in a container, kit, pack, or dispenser together with instructions for administration.

The nucleic acid molecules of the invention can be inserted into vectors and used as gene therapy vectors. Gene therapy vectors can be delivered to a subject by, for example, intravenous injection, local administration (see, *e*. *g*., U. S. Patent No. 5,328, 470) or by stereotactic injection (see, *e*. *g*., Chen, et al., 1994. Proc. Natl. Acad. Sci. USA 91 :3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e. g*., retroviral vectors, the pharmaceutical preparation can include one or more cells that produce the gene delivery system.

Accordingly, another aspect of the invention pertains to vectors, preferably expression vectors, containing a nucleic acid encoding a fused polypeptide of the invention, or derivatives, fragments, analogs or homologs thereof. As used herein, the term "vector" refers to a nucleic acid molecule capable of transporting another nucleic acid to which it has been linked. One type of vector is a "plasmid", which refers to a linear or circular double stranded DNA loop into which additional DNA segments can be ligated. Another type of vector is a viral vector, wherein additional DNA or RNA segments can be ligated into the viral genome. Certain vectors are capable of autonomous replication in a host cell into which they are introduced (*e*. *g*., bacterial vectors having a bacterial origin of replication and episomal mammalian vectors; yeast vectors). Other vectors (*e. g*., non-episomal mammalian vectors) are integrated into the genome of a host cell upon introduction into the host cell, and thereby are replicated along with the host genome.

Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e. g*., replication defective retroviruses, adenoviruses and adeno-associated viruses), which serve equivalent functions. Additionally, some viral vectors are capable of targeting a particular cells type either specifically or non-specifically.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively linked to the nucleic acid sequence to be expressed. Within a recombinant expression vector, "operably linked" is intended to mean that the nucleotide sequence of interest is linked to the regulatory sequence (s) in a manner that allows for expression of the nucleotide sequence (*e. g*., in an in vitro transcription/translation system or in a host cell when the vector is introduced into the host cell). The term "regulatory sequence" is intended to include promoters, enhancers and other expression control elements (*e. g*., polyadenylation signals). Such regulatory sequences are described, for example, in Goeddel; GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e. g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e. g*., chimeric polypeptides, mutant forms of the chimeric polypeptide, fusion proteins, etc.).

The recombinant expression vectors of the invention can be designed for expression of the chimeric polypeptide in prokaryotic or eukaryotic cells. For example, the chimeric polypeptide can be expressed in bacterial cells such as *E*. *coli,* insect cells (using baculovirus expression vectors) yeast cells, plant cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated in vitro, for example using T7 promoter regulatory sequences and T7 polymerase. Expression of proteins in prokaryotes is most often carried out in *E*. *coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (1) to increase expression of recombinant protein; (2) to increase the solubility of the recombinant protein; and (3) to aid in the purification of the recombinant protein by acting as a ligand in affinity purification. Often, in fusion expression vectors, a proteolytic cleavage site is introduced at the junction of the fusion domain and the recombinant protein to enable separation of the recombinant protein from the fusion domain subsequent to purification of the fusion protein. Such enzymes, and their cognate recognition sequences, include Factor Xa, thrombin and enterokinase. Typical fusion expression vectors include pGEX (Pharmacia Biotech Inc ; Smith and Johnson, Gene 67: 31-40 (1988)), pMAL (New England Biolabs, Beverly, Mass.) and pRIT5 (Pharmacia, Piscataway, N. J.) that fuse glutathione S-transferase (GST), maltose E binding protein, or protein A, respectively, to the target recombinant protein.

Examples of suitable inducible non-fusion *E*. *coli* expression vectors include pTrc (Amrann et al., Gene 69: 301-315 (1988)) and pET 1ld (Studier et al., GENE EXPRESSION TECHNOLOGY: METHODS IN ENZYMOLOGY 185, Academic Press, San Diego, Calif. 60-89 (1990)).

One strategy to maximize recombinant protein expression in *E*. *coli* is to express the protein in a host bacteria with an impaired capacity to proteolytically cleave the recombinant protein. See, Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E*. *coli* (Wada et. al., Nucleic Acids Res. 20: 2111-2118 (1992)). Such alteration of nucleic acid sequences of the invention can be carried out by standard DNA synthesis techniques.

In another embodiment, the chimeric polypeptide expression vector is a yeast expression vector. Examples of vectors for expression in yeast *S. cerevisiae* are well known in the art.

Alternatively, the chimeric polypeptide can be expressed in insect cells using baculovirus expression vectors.

In yet another embodiment, a nucleic acid of the invention is expressed in mammalian cells using a mammalian expression vector. When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cell, see, *e. g*., Chapters 16 and 17 of Sambrook et al., MOLECULAR CLONING: A LABORATORY MANUAL. 3rd., Cold Spring Harbor Laboratory, Cold Spring Harbor Laboratory Press, Cold Spring Harbor, N. Y., (2001).

In another embodiment, the recombinant mammalian expression vector is capable of directing expression of the nucleic acid preferentially in a particular cell type (*e. g*., tissue-specific regulatory elements are used to express the nucleic acid). Tissue-specific regulatory elements are known in the art.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein. Additionally, host cells could be modulated once expressing the chimeric polypeptide, and may either maintain or loose original characteristics.

A host cell can be any prokaryotic or eukaryotic cell. For example, chimeric polypeptide can be expressed in bacterial cells such as *E*. *coli,* insect cells, yeast or mammalian cells (such as Chinese hamster ovary cells (CHO) or COS cells). Alternatively, a host cell can be a premature mammalian cell, *i*. *e*., pluripotent stem cell. A host cell can also be derived from other human tissue. Other suitable host cells are known to those skilled in the art.

Vector DNA can be introduced into prokaryotic or eukaryotic cells via conventional transformation, transduction, infection or transfection techniques. As used herein, the terms "transformation" "transduction", "infection" and "transfection" are intended to refer to a variety of art-recognized techniques for introducing foreign nucleic acid (*e. g*., DNA) into a host cell, including calcium phosphate or calcium chloride coprecipitation, DEAE-dextran-mediated transfection, lipofection, or electroporation. In addition transfection can be mediated by a transfection agent. By "transfection agent" is meant to include any compound that mediates incorporation of DNA in the host cell, *e. g*., liposome. Suitable methods for transforming or transfecting host cells can be found in Sambrook, et al. (MOLECULAR CLONING: A LABORATORY MANUAL. 3rd, Cold Spring Harbor Laboratory, Cold Spring Harbor Laboratory Press, Cold Spring Harbor, N. Y. , (2001)), and other laboratory manuals.

Transfection may be "stable" (*i*. *e*. intergration of the foreign DNA into the host genome) or "transient" (*i. e*., DNA is episomally expressed in the host cells).

For stable transfection of mammalian cells, it is known that, depending upon the expression vector and transfection technique used, only a small fraction of cells may integrate the foreign DNA into their genome the remainder of the DNA remains episomal. In order to identify and select these integrants, a gene that encodes a selectable marker (*e*. *g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding or can be introduced on a separate vector. Cells stably transfected with the introduced nucleic acid can be identified by drug selection (*e*. *g*., cells that have incorporated the selectable marker gene will survive, while the other cells die).

### BRIEF DESCRIPTION OF THE DRAWINGS

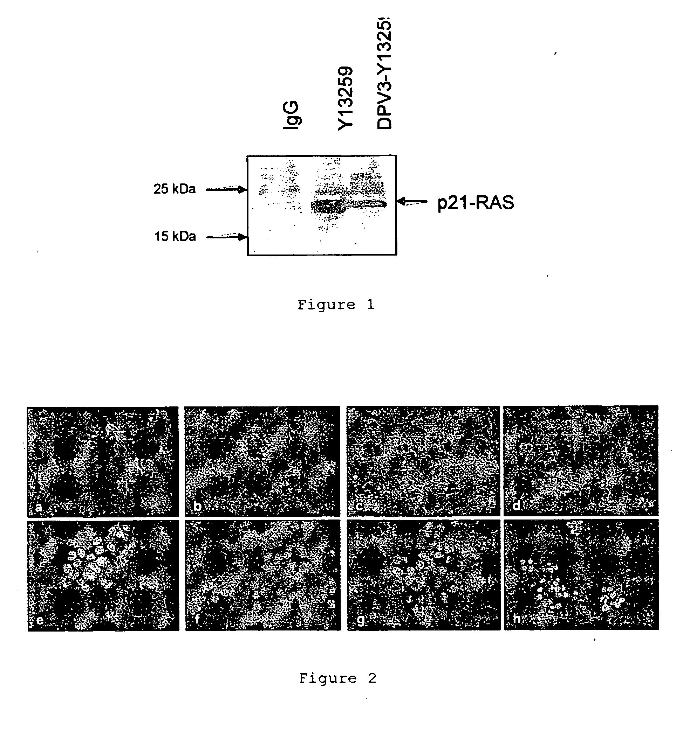

Figure 1 shows immunodetection of p21-RAS protein in MCF7-RAS cellular extracts using Y13259 antibody, DPV3-Y13259 or a non specific rat IgG antibody, as primary antibodies and an anti rat-HRP as secondary antibody for immunodetection. Data shown are representative of three independent experiments.
Figure 2 shows intracellular delivery of Y13259 alone or conjugated to DPV3 or DPV15b. HCT 116 human cancer cells were incubated with 50 µg/ml of DPV3-Y13259, DPV15b-Y13259 or Y13259 antibody for 4 hours at 37°C and then fixed in methanol/acetone prior immunofluorescence staining. Immunostaining of Y13259 antibody was performed using an anti rat-TRITC. Untreated cells (as internal negative control) (a), Y13259 (b), DPV3-Y13259 (c) or DPV15b-Y13259 treated cells (d); DAPI staining (e, f, g, h) shows nuclei for each cited conditions.

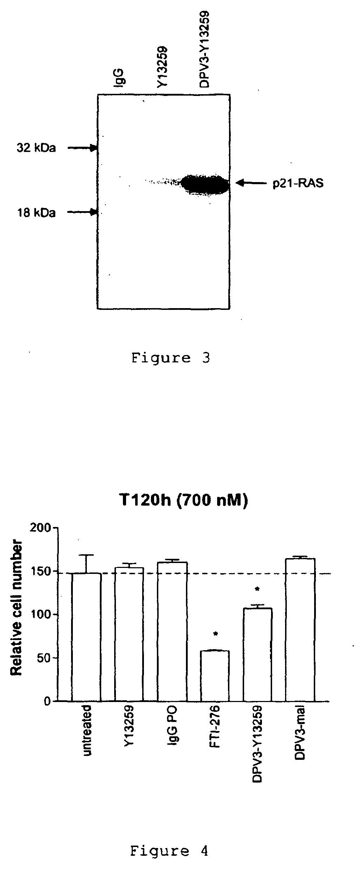

Figure 3 shows immunoprecipitation experiments performed on MCF7-RAS cells that demonstrate intracellular delivery of active Y13259 antibody by DPV3. Cells were incubated with 50 µg/ml of rat IgG as non specific antibody, Y13259 or DPV3-Y13259 for 4 hours at 37°C. Total protein lysates were immunoprecipited with protein G Sepharose, loaded on SDS-PAGE and immunoblotted with the pan RAS antibody. Data shown are representative of three independent experiments.
Figure 4 shows inhibitory effect of DPV3-Y13259 on *K*-RAS^{mt} HCT 116 cell proliferation. Cells were incubated for 120 hours with the antibody alone (Y13259; 700 nM), or the DPV3-Y13259 conjugate (700 nM), with DPV3-maleimide as internal controls (DPV3-mal; 700 nM). Non-relevant IgG1 anti-peroxydase immunoglobulins (IgG PO; 700 nM) and an inhibitor of RAS farnesyltransferase (FTI-276; 100 nM) were used as internal controls. Results are expressed as mean ± SEM of three independent experiments performed in duplicate. * p<0.05 vs not treated cells.

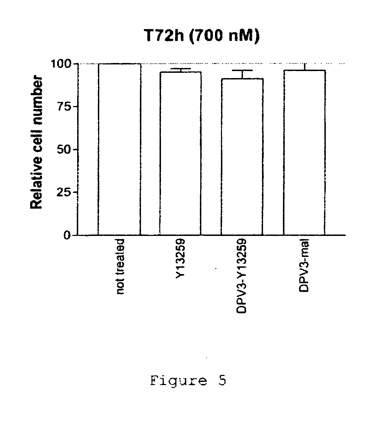

Figure 5 shows lack of effect of DPV3-Y13259 on wild-type RAS HT-29 cell proliferation. Cells were incubated for 72 hours with the antibody alone (Y13259; 700 nM), or once conjugated to DPV3 (DPV3-Y13259 conjugate; 700 nM), with DPV3-maleimide as internal controls (700 nM). Results are expressed as mean ± SEM of two independent experiments performed in duplicate.

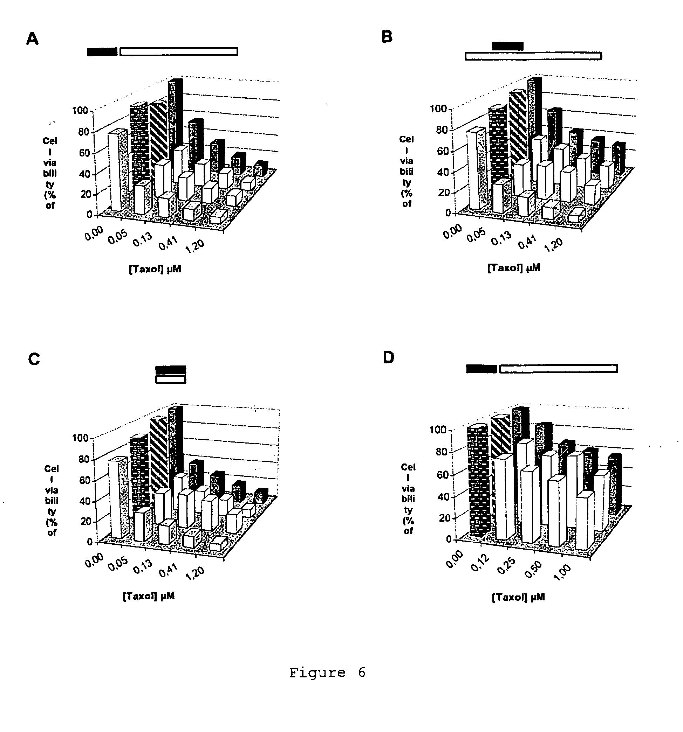

Figure 6 shows that DPV3-Y13259 can enhance Taxol®-induced apoptosis in HCT 116 (RAS activated) cells but not in HT-29 (wt RAS) cells. Cells were exposed to increasing concentrations of Taxol® for 4 hours followed by removal of media and incubation for further 72 hours; DPV3-Y13259 was combined to Taxol® following different protocols. (A) HCT 116 cells were incubated with Taxol® for 4 hours before addition of DPV3-Y13259 for further 72 hours. (B) DPV3-Y13259 was added to the cultured HCT 116 cells 24 hours prior Taxol® short incubation (4 hours). Medium was removed and cells were incubated again with DPV3-Y13259. No effect was observed when HCT 116 were incubated with DPV3-Y13259 and Taxol® altogether for 4 hours (C) as well as when wild-type RAS HT-29 cells were treated with Taxol® for 4 hours followed by removal of media and incubation for further 72 hours (D). Data show mean ± SEM of 3 independent experiments.

### EXAMPLES

Other advantages and characteristics of the invention will appear from the following examples which refer to the above figures. The examples are given to illustrate the invention but not to limit the scope of the claims.

### I- Materials and Methods

### I-1 Products

- Y13259: Rat monoclonal antibody anti-RAS (IgG1 isotype; Hamer et al., Hybridoma 9, 573-587 (1990) obtained from Y13259 hybridoma clone production (Serotec) (B lymphocytes; ATCC# CRL-1742). MW= 150 kDa.
- Normal rat IgG (santa Cruz # sc-2026): used as negative controls for Western blotting and immunoprecipitation applications.
- Cell-penetrating peptides:
   DPV3 (SEQ ID NO 2) that further contains a cysteine (Cys) at the C terminal position of the amino acid sequence
   DPV15b (SEQ ID NO 12) that further contains a cysteine (Cys) at the N terminal position of the amino acid sequence
- DPV3-Y13259 and DPV15b-Y13259: Conjugations of cell-penetrating peptide DPV3 or cell-penetrating peptide DPV15b to Y13259 were performed chemically (see Example I-3-a). Between two and three cell-penetrating peptides are bound per IgG. MW= 150 kDa.
- DPV3-maleimide and DPV15b-maleimide (a maleimide group has been conjugated to the terminal cysteine of DPV3 and DPV 15b)
- FTI-276 (Calbiochem # 344550): a highly potent and selective peptidomimetic of the carboxyl terminal of RAS proteins. Inhibitor of famesyltransferase (FTase) *in vitro,* (MW= 433.6). It has been shown to block the growth of a human lung carcinoma expressing K-RAS in nude mice.
- Mouse monoclonal IgG2b pan RAS antibody (clone F132 from Santa Cruz # sc-32) recognizes all mutated RAS forms - used for immunoprecipitation study.
- Rhodamine (TRITC) -conjugated affiniPure Donkey anti-rat IgG (Jackson ImmunoResearch # 712-025-150) used for immunocytochemistry.
- Peroxydase-conjugated affinity purified anti-rat IgG (TEBU # 612-1302) used for Western blotting immunodetection and as negative controls for cell viability assays.
- Protein G sepharose (Amersham # 17061801) for immunoprecipitation applications.
- Taxol®/Paclitaxel: (Hauser Lab). MW=853.92 g/mol. Stock dilution in Cremophore 20%, stored at room temperature.

### I-2 Cell lines

- HCT 116: epithelial cells from human colon carcinoma, express mutated Ki-RAS. Origin: ATCC # CCL-247. Cells were cultured in Mc Coy's 5a medium + 1.5 mM L-glutamine + 10% FCS.
- MCF7-RAS: epithelial cells from human breast carcinoma, transfected with v-H-RAS. Origin: G. Perret (Bobigny, France). Cells were cultured in DMEM + 10% FCS + 1.5 mM L-glutamine.
- HT-29: human epithelial cells from human colon adenocarcinoma, express wild-type RAS. Origin: ATCC # HTB-38. Cells were cultured in Mc Coy's 5a medium + 10% FCS + 1.5mM L-glutamine.

### 1-3-a Chemical Conjugation of CPP to antibody

A two-step conjugation strategy was used for the chemical conjugation of a CPP (*e.g*., DPV3 and DPV15b) onto an antibody. The first step was the activation of antibody with the heterobifunctional cross-linking reagent Succinimidyl-4-(N-maleimidomethyl)cyclohexane-1-carboxylate (SMCC). This heterobifunctional linker reacted with available lysine amines of the antibody. After elimination of excess cross-linking reagent by dialfiltration (Viaspin®, cut off 3000 Daltons, the CPP was then added, its thiol function (N- or C-terminal cysteine) reacted with the maleimide moiety of the SMCC. The cross-linked conjugates were analysis by SDS/PAGE (12%) - Coomassie blue R-250 staining and MALDI-TOF.

Examples of coupling between a CPP and an antibody can be found in PCT Patent Application n° WO 01/64738 and WO 05/016960.

### I-3-b Chemical Conjugation of CPP with maleimide

A one-step conjugation strategy was used for the chemical conjugation of maleimide to CPP, in order to generate a CPP control (*e.g*., DPV3-maleimide and DPV15b-maleimide). This conjugation was undertaken in DMF with an excess of maleimide and masked accessible thiol functions on the CPP. After 30 min of incubation, the excess of maleimide was remove by 3 extractions with water/Dichloromethane. The conjugate (in aqueous layer) was then lyophilized

### 1-4 Western blot analysis

Proteins (50 µg of total cell lysates in RIPA buffer supplemented with proteases inhibitors) were loaded on a SDS-PAGE according to Laemmli, electroblotted onto nitrocellulose membrane. Immunoreactive proteins were detected after immunoblotting either with Y13259 or DPV3-Y13259 antibodies (50 µg/ml).

### 1-5 Immunoprecipitation

Cells were incubated with 50 µg/ml of either normal rat IgG (negative control) or non-conjugated Y13259 or DPV3-Y13259 conjugate for 4 hours at 37°C, then trypsinized (to remove material attached to cell surface), lysed, incubated with portein G Sepharose, transferred on PVDF (Polyvinylidene Difluoride) membrane and blotted with mouse monoclonal Pan-RAS.

### I-6 In vitro internalization studies

Cells in complete culture medium were incubated for 4 hours at 37°C with either CPP-Y13259 conjugates or non-conjugated Y13259 antibody at 50 µg/ml, then rinsed in phosphate buffer and fixed in methanol/acetone 5 min and permeabilized in phosphate buffer containing 0.25% Triton X100 and 1% BSA. Cells were labelled with an anti-rat-TRITC antibody and counterstained with DAPI (4',6-diamidino-2-phenylindole). Cells were mounted in slow fade light antifade solution with DAPI and analyzed by fluorescence microscopy.

### I-7 In vitro efficacy on human carcinoma cell lines

HCT 116 and HT-29 were seeded at 8 x10³ cells/well (96-well plates) in medium + 0.5% and 10% FCS (Foetal Calf Serum) respectively. The day after, cells were treated with increasing concentrations of tested compounds in complete medium (+ 10% FCS) and cells were counted overtime at 24h, 48h, 72h and 120h using the WST-1 method, described hereafter. The medium was removed and 100 µl of fresh medium without FCS supplemented with 10% WST-1 solution was added on cells for 2h at 37°C, 5% CO₂. Measurement of A₄₅₀ₙₘ - A₆₉₀ₙₘ (blank = 0 cells) was performed to determine cell number.

### I-8 Viability assays

Viability assays were performed on adherent cells as following: cells were seeded in 96-well plates 24 hours before treatment. To define the best schedule for the combination of the two drugs, either simultaneous or sequential exposures were tested. According to the expected biologic effects, and the clinical efficacy, toxicity, and pharmacodynamic data already available, a short time-exposure for Taxol® and a long time-exposure for DPV3-Y13259 were performed, in 4 different combination schedules as described below.

### Protocol #1: Sequential schedule

Cells were exposed to Taxol® or vehicle for 4 h, washed and subsequently incubated in media containing DPV3-Y13259 for 72 h.

### Protocol #2: Reverse sequential schedule

Cells were treated with DPV3-Y13259 or vehicle for 24 h. Then, cells were exposed to Taxol® for 4 h and then washed off followed by DPV3-Y13259 treatment continued until 72 h.

### Protocol #3: Continuous combination, short time

Cells were placed in medium containing both Taxol® and DPV3-Y 13259 or vehicle and were incubated for 4 h, exposing cells to both agents continuously.

Cells were treated according to the different protocols. Cell viability was then analyzed using the WST-1 assay and IC₅₀ values (molar concentration that inhibits 50% of cell viability) were estimated from sigmoid regression, using Graph Pad Prism 3.02 software.

### I-9 Statistical analyses

Data are shown as mean ± standard error of the mean (SEM) of n distinct observations. Statistical comparisons were analyzed with ANOVA followed by *Tukey's post hoc* test. In all cases, a threshold value of p<0.05 was considered as a minimum level of significance. Statistical analyses were performed using Graph Pad Prism 3.02 software and CalcuSyn for dose effect analysis.

### I-10 Data analysis for the combination treatments

The terms synergy and antagonism are frequently used in different ways to describe the effect of combining two or more drugs in either cell lines, tumour-bearing mice or the clinic. In cell culture experiments, synergy and antagonism refer to growth inhibition/cytotoxicity that is greater or lesser than predicted from independent drug action, respectively. This arises when one drug modulates the activity of the second drug by, for example, affecting drug uptake, metabolism, cell cycle phase distribution, target/pathway inhibition or changing the threshold for apoptosis. Additivity is the result of two or more drugs working independently with both contributing equally to activity. Various mathematical models are available including isobolograms, surface response models and the median-effect analysis of Chou and Talalay. Effects of multidrugs treatment on cell viability were analyzed according to the computerized method of Chou and Talalay (Adv Enzyme Regul., 22, 27-55 (1984)). The range of Combination Index (CI) and the symbols are defined from those described earlier by Chou. CI < 1, = 1 and > 1 indicate synergism, additive effect and antagonism, respectively. See Table 2 below:

**Table2: Recommended symbols for describing synergism or antagonism in drug combination studies analyzed with the combination index method**

| Range of Combination Index (CI) | Symbol | Description |
|---|---|---|
| <0.1 | +++++ | Very strong synergism |
| 0.1-0.3 | ++++ | Strong synergism |
| 0.3-0.7 | +++ | Synergism |
| 0.7-0.85 | ++ | Moderate synergism |
| 0.85-0.9 | + | Slight synergism |
| 0.9-1.10 | ± | Nearly additive |
| 1.10-1.20 | - | Slight antagonism |
| 1.20-1.45 | -- | Moderate antagonism |
| 1.45-3.3 | --- | Antagonism |
| 3.3-10 | ---- | Strong antagonism |
| >10 | ----- | Very strong antagonism |

### II- Intracellular delivery of Y13259 using the cell-penetrating peptide DPV3 and DPV 15b

The purpose of this study was to internalize Y13259 anti-RAS antibody using a cell-penetrating peptide to specifically target the mutated RAS signalling. Different cell lines were treated with Y 13259 chemically attached to a CPP enabling cell membrane penetration to determine whether CPP-anti-RAS antibody conjugates can selectively block the growth of RAS-transformed cancer cells.

### II-1 Y13259 binding to RAS protein

In order to check that CPP conjugation did not modify Y13259 affinity for RAS, Western blot analysis was performed with total cellular extracts from MCF7-RAS. DPV3-Y13259 binding to RAS was compared to that of the free antibody. As shown in Figure 1, DPV3-Y13259 conjugate is able to recognize its target RAS.

### II-2 Qualitative analysis of CPP-Y13259

DPV3-Y13259 and DPV15b-Y13259 were incubated on HCT 116 cells for 4 hours at 37°C. As shown in Figure 2, DPV3 allows IgG internalization in the cytoplasm (Figure 2c and 2g). Previous studies reported that DPV3 and DPV15b linked to IgG anti peroxydase were localized in the cytoplasm or in the nucleus, respectively. However, DPV15b-Y13259 conjugates were also localized in the cytoplasm (Figure 2d and 2h). This data demonstrate that affinity of the anti-RAS antibody for its cytoplasmic target is determinant for the accumulation site of the CPP conjugate.

### II-3- Intracellular delivery of Y13259 by DPV3

Internalization with Y13259, DPV3-Y13259 and rat IgG was performed on MCF7-RAS cells for 4 hours; cells were harvested with trypsine (this step removes cell surface bound CPP-antibody) and lysed in RIPA buffer for RAS analysis by Western blot. As shown in Figure 3, DPV3-Y13259 is internalized and able to immunoprecipitate the RAS protein, whilst naked Y13259 or non relevant IgG do not, suggesting that DPV3 enables intracellular delivery of a whole active antibody. DPV3-Y13259 binding to RAS could occur during the internalization step (showing that the conjugate went to the cytosol fraction).

### II-4 In vitro anti-proliferative activity

CPP-Y13259 conjugates were tested in a cell proliferation assay on mutated RAS-expressing and wild-type RAS expressing cell lines (HCT 116 and HT-29, respectively). FTI-276, a famesyltransferase inhibitor that inhibits RAS-mediated cell proliferation constituted a positive control. For HCT 116, cells were treated with increasing concentrations of CPP conjugates (up to 700 nM) in complete medium (10% FCS) from 0 to 120 h.

As shown in Figure 4, FTI-276 inhibited HCT 116 cell proliferation; its effect lasted at least 120 hours. As suggested by internalization and immunoprecipitation studies, rat IgGs (Y13259 or anti peroxydase) were not able to cross the membrane by themselves and no inhibitory effect on growth rate could be detected. Y13259 and CPPs-maleimide showed no inhibitory effect on HCT 116 cell proliferation, whilst the CPP-Y13259 conjugates down-regulated cell proliferation. This effect was observed at 72 hours after drug exposure and lasted at least 120 hours (maximum time tested). Interestingly, Y13259 conjugate that is linked to a "cytoplasmic" CPP (DPV3) (De Coupade et al., (Biochem J. (2005)) resulted in a better inhibition than the one linked to a "nuclear" CPP (DPV15b) (De Coupade et al., (Biochem J. (2005)), (Figure 4). Efficient concentrations for DPV3-Y13259 ranged from 85 to 700 nM whereas lower concentrations were without any effect (data not shown).

Lack of activity of DPV3-Y13259 in wild-type RAS expressing cells after 72 hours of continuous exposure was observed (Figure 5). This result shows the specificity of action of the delivered antibody. In this experiment, DPV15b was not tested since it was less active than DPV3 in HCT 116 model.

### II-5 Conclusion

This study showed that a cell-penetrating peptide (*e.g*., DPV3 or DPV15) could deliver intracellularly a whole antibody (150 kDa) without altering its biochemical properties (affinity or cellular localization). In terms of biological activity, CPP-Y13259 conjugates reduced the growth rate of mammalian cancer involving mutated RAS cells but not their counterpart (wild-type) cells. Once conjugated to Y13259, DPV3 peptide, reported as a highly efficient cell penetrating peptide for cytoplasmic delivery of a cargo resulted in a greater inhibitory effect than DPV15b.

### III- In vitro evaluation of combined treatment with DPV3-Y13259 and Taxol® in tumor cell line in culture

The purpose of this study was to further investigate the influence of the two entities, DPV3-Y13259 and Taxol®, separately and in combination, on cancer cell proliferation. The rationale for combining two or more therapeutic agents is to achieve lower drug doses, reduce toxicity and minimize or delay the emergence of resistance by target cells, and to identify potential synergistic effect. The aim was to investigate 1) the potential for DPV3-Y13259 to enhance the cytotoxicity of Taxol® based antitumor agents, and 2) which was the best possible combination schedules.

### III-1 Viability assays

Figure 6 presents results obtained with different concentrations of DPV3-Y13259 in combination with Taxol®. As described in Material and Methods section, 3 protocols were tested.

Cell viability data obtained in presence of Taxol® indicate that combine treatment with DPV3-Y13259 treatment induces a dose-dependent decrease in HCT 116 cell viability for protocols 1 and 2 (Figures 6A and 6B). Taxol® is known to disrupt equilibrium between free tubulin and microtubules causing stabilization of cytoplasm microtubules. Therefore Taxol® treatment prior DPV3-Y13259 incubation could affect endocytosis of the CPP conjugate. However, no significant differences were observed between protocol 1 and 2. No effect of DPV3-Y13259 on Taxol® cytotoxicity was observed for protocol 3 (Figure 6C). This lack of effect may be due to the short incubation time with DPV3-Y13259.

As expected, no effect of DPV3-Y13259 on HT-29 cells, expressing wild-type RAS protein, proliferation was observed with protocol 1 (Figure 6D).

### III-2 Analysis of multiple drug effects

The multiple drug effect analysis of Chou and Talalay, which is based on the median effect principle (Chou and Talalay, Adv Enzyme Regul., 22: p. 27-55 (1984) ; Chou et al.., J Natl Cancer Inst,. 86(20): p. 1517-24 (1994)) was used to examine the nature of the interaction observed between DPV3-Y13259 and Taxol®. To determine whether the increased effect on cell viability observed after combinatorial treatment of HCT 116 cells with DPV3-Y13259 and Taxol® in comparison to separate treatment, corresponds to additive or synergistic effects, a detailed analysis of multiple drug effects was undertaken according to Chou and Talalay's method. The calculated dose effect parameters CI are given in Table 3 below:

**Table 3. Mutually non-exclusive CI for experimental values. Dose-effect relationship parameters for Taxol® and DPV3-Y13259 in HCT 116 lung cancer cells. CI < 1, = 1 and > 1 indicate synergism, additive effect or antagonism, respectively.**

| Taxol® (µM) | DPV3-Y13259 (µg/ml) | CI protocol 2 | | CI protocol 1 | |
|---|---|---|---|---|---|
| 0.045 | 200 | 0.130 | ++++ | 0.521 | +++ |
| 0.045 | 100 | 0.111 | ++++ | 0.387 | +++ |
| 0.045 | 20 | 0.164 | ++++ | 0.557 | +++ |
| 0.13 | 200 | 0.335 | +++ | 0.624 | +++ |
| 0.13 | 100 | 0.243 | ++++ | 0.543 | +++ |
| 0.13 | 20 | 0.365 | +++ | 0.394 | +++ |
| 0.41 | 200 | 0.836 | ++ | 0.772 | ++ |
| 0.41 | 100 | 0.498 | +++ | 0.603 | +++ |
| 0.41 | 20 | 0.866 | + | 0.488 | +++ |
| 1.2 | 200 | 1.109 | - | 0.783 | ++ |
| 1.2 | 100 | 1.738 | --- | 0.950 | +/- |
| 1.2 | 20 | 1.277 | -- | 0.733 | ++ |

According to Chou and Talalay analysis, DPV3-Y13259 and Taxol® synergize to induce decreased cell viability in HCT 116 cells. The synergistic effect is especially prominent at low Taxol® dosages and was independent of the treatment schedule. The combination index is between 0.1 and 0.85 for the lowest Taxol® concentration. For the strongest Taxol® concentration, above 1.2 µM, the combination index is superior to 1 with protocol 2. The synergistic effect is prominent at lower Taxol® concentration (<1.2 µM) especially with the protocol 2.

### III-3 Conclusion

DPV3-Y13259 combined with Taxol® induces cytotoxicity in a synergistic manner in colon cancer cells. Transferred to the *in vivo* studies, the combination of DPV3-Y13259 with Taxol® might allow a reduction in Taxol® doses. In summary, these data show that DPV3-Y13259 may be usefully combined with other cytotoxic drugs such as paclitaxel.

### IV Evaluation of the antitumor activity of DPV3-Y13259 in combination with Paclitaxel on a HCT 116 colon carcinoma xenograft model: proposed study

The aim of this proposed study is to test the efficacy of DPV3-Y13259 in combination with a suboptimal dose of paclitaxel in the HCT 116 colon carcinoma xenograft model in nude mice. The suboptimal dose of paclitaxel can be easily determined by one skilled in the art. The Applicant has found it 5 mg/kg following a Q7D1x3W administration schedule (intra venous administration).
Therapeutic agents: Y13259, DPV3-Y13259, and Paclitaxel (PTX)
Animals: athymic nu/nu NMRI-nude mouse strain.
Tumor model: HCT 116 colon carcinoma model can be selected because this cell line expresses a constitutive activated form of *ras* oncogen: K-*ras.*
Tumor induction: cells are thawed in a 75 cm² flask and amplified. After amplification, the cells are suspended in DMEM medium (1 x 10⁷ cells/100 µL). The HCT 116 tumors are established by intradermal injection of 100 µL of the cell suspension in the right flank of mice using 1ml Terumo syringes with 26G1/2-0.45x12 mm needles (Polylabo) following the procedure FD 31/10/01 V1.

DPV3-Y13259, Y13259 and paclitaxel solutions:
The DPV3-Y13259 conjugate and Y13259 are deluted in PBS, 0.3M NaCl in order to administer the adequate dose.
Clinical grade Paclitaxel is diluted in 0.9% NaCl in order to administer the adequate dose.

Mice are treated by bolus intravenous injection following a Q3D2x3W (2 weekly injections for 3 weeks) or Q7D1x3W (1 weekly injection for 3 weeks) schedule. Injections are performed when the tumors reach a size of about 100 mm³. DPV3-Y13259 doses are expressed in mg equivalent of Y13259 per kg. The groups are:

| Vehicle (NaCl and NaCl ethanol/cremophor) Q3D2x3W and Q7D1x3W respectively | | | | |
|---|---|---|---|---|
| DPV3-Y13259 | 40 mg/kg | Q3D2 3 W | PTX 5 mg/kg | Q7D1x3W |
| DPV3-Y13259 | 20 mg/kg | Q3D2 3W | PTX 5 mg/kg | Q7D1x3W |
| DPV3-Y13259 | 40 mg/kg | Q3D2 3W | | |
| DPV3-Y13259 | 20 mg/kg | Q3D2 3W | | |
| DPV3-Y13259 | 10 mg/kg | Q3D2 3W | | |
| Y 13259 | 40 mg/kg | Q3D2 3W | PTX 5 mg/kg | Q7D1x3W |
| Y13259 | 40 mg/kg | Q3D2 3W | | |
| PTX | 5 mg/kg | Q7D1x3W | | |
| PTX | 15 mg/kg | Q7D1x3W | (positive control) | |

### Data analysis:

Body weight, tumor volume and survival parameters are monitored 3 times a week. Tumor volumes are determined from caliper measurements, [length x (width)²/2]. Each individual mouse is sacrificed when its tumor reached approximately 1500 mm³ or if the animal showed any signs of pain like hunching, convulsions, 25% body weight loss for 2 consecutive measurements or 30% body weight loss at one measurement. All those parameters are reported. Mortality of animals is recorded every day except on the weekend.
Treated / Control value (T/C) is the ratio of the mean tumor volume of treated animals versus the mean tumor volume of control animals, expressed as a percentage.
The statistical analyses of tumor growth and survival parameters (Dunnett and LogRank tests, respectively) are assessed weekly using the GraphPad prism 3.02 software.

## Claims

1. A chimeric polypeptide **characterized in that** said polypeptide comprises a first domain and a second domain, wherein
- the first domain comprises an amino-acid sequence facilitating active transport across a biological membrane by the binding to an glycosaminoglycan, which is selected from the group consisting of a) (XBBBXXBX)n ; b) (XBBXBX)n ; c) (BBXmBBXp)n ; d) (XBBXXBX)n ; e) (BXmBB)n ; f) (BmXX)n or g) an antibody fragment, wherein each B is independently a basic amino acid preferably lysine or arginine; each X is independently a non-basic amino acid preferably hydrophobic amino acid; each m is independently an integer from zero to five; each n is independently an integer between one and ten; and each p is independently an integer between zero to five; and
- the second domain comprises an anti-activated RAS neutralizing antibody, fragment or derivative thereof, which specifically binds to an epitope on the activated RAS protein.

2. The chimeric polypeptide according to claim 1, wherein the first domain comprises an amino-acid sequence facilitating active transport across a biological membrane, having a length of more than 4 amino acids, preferably more than 6 amino acids, and most preferably more than 13 amino acids.

3. The chimeric polypeptide according to any one of claims 1 or 2, wherein the first domain comprises an amino-acid sequence facilitating active transport across a biological membrane, having a length of less than 500 amino acids, preferably less than 35 amino acids, and most preferably less than 25 amino acids.

4. The chimeric polypeptide according to any one of claims 1 to 3, wherein each X is independently alanine, glutamic acid, isoleucine, leucine, methionine, phenylalanine, serine, tryptophan, valine, tyrosine or citrulline.

5. The chimeric polypeptide according to any one of claims 1 to 4, wherein the first domain comprises an amino acid sequence selected from the group comprising SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12.

6. The chimeric polypeptide according to claim 1, wherein the first domain comprises the amino acid sequence (BmXX)n; and wherein B is any basic amino acid; X is any non-basic amino acid; each m is independently a integer between 1 and 10; each n is independently a integer between 1 and 4.

7. The chimeric polypeptide according to claim 6, wherein the first domain comprises the amino acid sequence BBBBBBXXBBBBBBXX; wherein B is independently a basic amino acid; X is independently a non-basic amino acid.

8. The chimeric polypeptide according to claim 7, wherein X is selected from the group comprising glutamic acid and serine.

9. The chimeric polypeptide according to claim 8, wherein the first domain comprises SEQ ID NO: 2.

10. The chimeric polypeptide according to any of the previous claims, wherein the anti-activated RAS neutralizing antibody, fragment or derivative thereof has the ability to bind to an activated RAS protein resulting in inhibition of the biological activity of said activated RAS protein.

11. The chimeric polypeptide according to claim 10, wherein the anti-activated RAS neutralizing antibody, fragment or derivative thereof is selected in the group comprising the rat monoclonal antibody Y13259 (produced by the hybridoma clone originated from ATCC, number CRL-1742), fragments and derivatives thereof.

12. The chimeric polypeptide according to any of the previous claims, wherein the anti-activated RAS protein is selected from the group comprising H-RAS, N-RAS, K-RAS A and K-RAS B.

13. The chimeric polypeptide according to any of the previous claims, wherein the anti-activated RAS neutralizing antibody, fragment or derivative thereof is conjugated at the N- or C-terminal end of the first domain.

14. The chimeric polypeptide according to claim 13, wherein the anti-activated RAS neutralizing antibody, fragment or derivative thereof is conjugated to the first domain via a linker.

15. The chimeric polypeptide according to claim 14 wherein the linker is succinimidyl 4-(N-maleimidomethyl)cyclohexane-1-carboxylate (SMCC) or N-maleimidobutylamine.

16. The chimeric polypeptide according to any of claims 13 or 14, wherein both domains are fused by genetic engineering.

17. A polynucleotide comprising a nucleic acid sequence encoding a polypeptide according to claim 16.

18. A composition comprising a chimeric polypeptide according to any of claims 1 to 16 or a polynucleotide according to claim 17 and optionally a pharmaceutically acceptable carrier.

19. A use of a chimeric polypeptide according to any of claims 1 to 16 or a polynucleotide according to claim 17 for the manufacture of a medicament for treating a cancer.

## Patentansprüche

1. Chimäres Polypeptid, **dadurch gekennzeichnet, dass** das besagte Polypeptid eine erste und eine zweite Domäne aufweist, in denen
- die erste Domäne eine Aminosäurensequenz enthält, die den aktiven Transport durch eine biologische Membran durch die Bindung an ein Glykosaminoglykan erleichtert, das aus der Gruppe gewählt wurde, die aus folgendem besteht a) (XBBBXXBX)n ; b) (XBBXBX)n ; c) (BBXmBBXp)n ; d) (XBBXXBX)n ; e) (BXmBB)n ; f) (BmXX)n oder g) ein Antikörperfragment, in dem jedes B unabhängig eine basische Aminosäure ist, vorzugsweise Lysin oder Arginin ; jedes X unabhängig eine nicht-basische Aminosäure ist, vorzugsweise eine hydrophobe Aminosäure ; jedes m unabhängig eine ganze Zahl zwischen 0 und 5 ist ; jedes n unabhängig eine ganze Zahl zwischen 1 und 10 ist; und jedes p unabhängig eine ganze Zahl zwischen 1 und 5 ist; und
- die zweite Domäne einen aktivierten anti-RAS neutralisierenden Antikörper, ein Fragment oder Derivat desselben enthält, das sich spezifisch an ein Epitop an dem aktivierten RAS-Protein bindet.

2. Chimäres Polypeptid gemäß Anspruch 1, in dem die erste Domäne eine Aminosäuresequenz enthält, die den aktiven Transport durch eine biologische Membran erleichtert und deren Länge 4 Aminosäuren, vorteilhafterweise 6 Aminosäuren und noch vorteilhafterweise 13 Aminosäuren übersteigt.

3. Chimäres Polypeptid gemäß Anspruch 1 oder 2, in dem die erste Domäne eine Aminosäuresequenz enthält, die den aktiven Transport durch eine biologische Membran erleichtert und deren Länge kürzer als 500 Aminosäuren, vorteilhafterweise kürzer als 35 Aminosäuren und noch vorteilhaftererweise kürzer als 25 Aminosäuren ist.

4. Chimäres Polypeptid gemäß einem beliebigen der Ansprüche 1 bis 3, in dem jedes X unabhängig ein Alanin, eine Glutaminsäure, ein Isoleucin, ein Methionin, ein Plenylalanin, ein Serin, ein Tryptophan, ein Valin oder ein Zitrullin ist.

5. Chimäres Polypeptid gemäß einem beliebigen der Ansprüche 1 bis 4, in dem die erste Domäne eine Aminosäuresequenz enthält, die innerhalb der folgenden Gruppe gewählt wird: SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO: 10, SEQ ID NO: 11, SEQ ID NO: 12.

6. Chimäres Polypeptid gemäß Anspruch 1, in dem die erste Domäne eine Aminosäuresequenz (BmXX)n enthält; und in der B eine basische Aminosäure ist; in der X eine nicht-basische Aminosäure ist; jedes m unabhängig eine ganze Zahl von 1 bis 10 ist; jedes n unabhängig eine ganze Zahl von 1 bis 4 ist.

7. Chimäres Polypeptid gemäß Anspruch 6, , in dem die erste Domäne eine Aminosäuresequenz BBBBBBXXBBBBBBXX enthält, in der B unabhängig eine basische Aminosäure ist; in der X unabhängig eine nicht-basische Aminosäure ist.

8. Chimäres Polypeptid gemäß Anspruch 7, in der X in der Gruppe zu der Glutaminsäure und Serin gehören, gewählt wird.

9. Chimäres Polypeptid gemäß Anspruch 8, in dem die erste Domäne SEQ ID NO: 2 enthält.

10. Chimäres Polypeptid gemäß einem beliebigen der vorangehenden Ansprüche, in dem der aktivierte anti-RAS neutralisierende Antikörper, ein Fragment oder Derivat desselben, die Fähigkeit besitzt, sich an ein aktiviertes RAS Protein zu binden, was zur Hemmung der biologischen Aktivität des besagten aktivierten RAS-Proteins führt.

11. Chimäres Polypeptid gemäß Anspruch 10, in dem der aktivierte anti-RAS neutralisierende Antikörper, ein Fragment oder Derivat desselben, in der Gruppe gewählt wird, zu der ein monoklonaler Antikörper der Ratte Y13259 gehört (vom Klon des Hybridom aus ATCC Nummer CRL-1742 hergestellt), Fragmente oder Derivate desselben.

12. Chimäres Polypeptid gemäß einem beliebigen der vorangehenden Ansprüche, in dem der aktivierte anti-RAS neutralisierende Antikörper innerhalb der Gruppe gewählt wird, zu der H-RAS, N-RAS, K-RAS A und K-RAS B gehören.

13. Chimäres Polypeptid gemäß einem beliebigen der vorangehenden Ansprüche, in dem der aktivierte anti-RAS neutralisierende Antikörper, ein Fragment oder Derivat desselben, am N- oder C-Terminal Ende der ersten Domäne konjugiert wird.

14. Chimäres Polypeptid gemäß Anspruch 13, in dem der aktivierte anti-RAS neutralisierende Antikörper, ein Fragment oder Derivat desselben, durch einen Linker konjugiert wird.

15. Chimäres Polypeptid gemäß Anspruch 14, in dem der Linker ein Succinimidyl 4-(N-Maleimidomethyl) Cyclohexan-1-Carboxylat (SMCC) oder N-Maleimidobutylamin ist.

16. Chimäres Polypeptid gemäß Anspruch 13 oder 14, in dem die beiden Domänen gentechnologisch fusioniert werden.

17. Polynukleotid mit einer Sequenz von Nukleinsäuren, die ein Polypetid gemäß Anpruch 16 kodieren.

18. Eine ein chimäres Polypeptid enthaltende Verbindung gemäß einem beliebigen der vorangehenden Ansprüche 1 bis 16 oder ein Polynukleid gemäß Anspruch 17 und wahlweise ein pharmazeutisch akzeptabler Hilfsstoff.

19. Verwendung eines chimären Polypeptiden gemäß einem beliebigen der vorangehenden Ansprüche 1 bis 16 oder eines Polynukleiden gemäß Anspruch 17 zur Herstellung eines Medikaments zur Krebsbehandlung.

## Revendications

1. Polypeptide chimérique **caractérisé en ce que** ledit polypeptide comprend un premier domaine et un second domaine, dans lequel
- le premier domaine comprend une séquence d'acides aminés facilitant le transport actif à travers une membrane biologique par la liaison à un glycosaminoglycane choisi dans le groupe consistant en a) (XBBBXXBX)n ; b) (XBBXBX)n ; c) (BBXmBBXp)n ; d) (XBBXXBX)n ; e) (BXmBB)n ; f) (BmXX)n or g) un fragment d'anticorps, dans lequel chaque B est indépendamment un acide aminé basique préférentiellement la lysine ou l'arginine ; chaque X est indépendant un acide aminé non-basique préférentiellement un acide aminé hydrophobe ; chaque m est indépendamment un nombre entier allant de 0 à 5 ; chaque n est indépendamment un nombre entier allant de 1 à 10 ; et chaque p est indépendamment un nombre entier allant de 0 à 5 ; et
- le second domaine comprend un anticorps neutralisant anti-RAS activée, un fragment ou un dérivé de celui-ci, qui se lie spécifiquement à un épitope sur la protéine RAS activée.

2. Polypeptide chimérique selon la revendication 1, dans lequel le premier domaine comprend une séquence d'acides aminés facilitant le transport actif à travers une membrane biologique, ayant une longueur supérieure à 4 acides aminés, préférentiellement supérieure à 6 acides aminés, et encore plus préférentiellement supérieure à 13 acides aminés.

3. Polypeptide chimérique selon la revendication 1 ou 2, dans lequel le premier domaine comprend une séquence d'acides aminés facilitant le transport actif à travers une membrane biologique, ayant une longueur inférieure à 500 acides aminés, préférentiellement inférieure à 35 acides aminés, et encore plus préférentiellement inférieure à 25 acides aminés.

4. Polypeptide chimérique selon l'une quelconque des revendications 1 à 3, dans lequel chaque X est indépendamment une alanine, un acide glutamique, une isoleucine, une méthionine, une phénylalanine, une sérine, un tryptophane, une valine ou une citrulline.

5. Polypeptide chimérique selon l'une quelconque des revendications 1 à 4, dans lequel le premier domaine comprend une séquence d'acides aminés choisie dans le groupe comprenant SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12.

6. Polypeptide chimérique selon la revendication 1, dans lequel le premier domaine comprend la séquence d'acides aminés (BmXX)n ; et dans laquelle B est un acide aminé basique ; X est un acide aminé non-basique ; chaque m est indépendamment un nombre entier allant de 1 à 10 ; chaque n est indépendamment un nombre entier allant de 1 à 4.

7. Polypeptide chimérique selon la revendication 6, dans lequel le premier domaine comprend la séquence d'acides aminés BBBBBBXXBBBBBBXX, dans laquelle B est indépendamment un acide aminé basique ; X est indépendamment un acide aminé non-basique.

8. Polypeptide chimérique selon la revendication 7, dans lequel X est choisi dans le groupe comprenant l'acide glutamique et la sérine.

9. Polypeptide chimérique selon la revendication 8, dans lequel le premier domaine comprend SEQ ID NO: 2.

10. Polypeptide chimérique selon l'une quelconque des revendications précédentes, dans lequel l'anticorps neutralisant anti-RAS activée, un fragment ou un dérivé de celui-ci, a la capacité de se lier à une protéine RAS activée résultant en l'inhibition de l'activité biologique de ladite protéine RAS activée.

11. Polypeptide chimérique selon la revendication 10, dans lequel l'anticorps neutralisant anti-RAS activée, un fragment ou un dérivé de celui-ci, est choisi dans le groupe comprenant un anticorps monoclonal de rat Y13259 (produit par l'hybridome provenant de ATCC, numéro CRL-1742), des fragments ou des dérivés de celui-ci.

12. Polypeptide chimérique selon l'une quelconque des revendications précédentes, dans lequel l'anticorps neutralisant anti-RAS activée est choisi dans le groupe comprenant H-RAS, N-RAS, K-RAS A et K-RAS B.

13. Polypeptide chimérique selon l'une quelconque des revendications précédentes, dans lequel l'anticorps neutralisant anti-RAS activée, un fragment ou un dérivé de celui-ci, est conjugué à l'extrémité N- ou C-terminale du premier domaine.

14. Polypeptide chimérique selon la revendication 13, dans lequel l'anticorps neutralisant anti-RAS activée, un fragment ou un dérivé de celui-ci, est conjugué par un lieur.

15. Polypeptide chimérique selon la revendication 14, dans lequel le lieur est succinimidyl 4-(N-maleimidomethyl) cyclohexane-1-carboxylate (SMCC) ou N-maleimidobutylamine.

16. Polypeptide chimérique selon la revendication 13 ou 14, dans lequel les deux domaines sont fusionnés par génie génétique.

17. Polynucléotide comprenant une séquence d'acides nucléiques encodant un polypeptide selon la revendication 16.

18. Composition comprenant un polypeptide chimérique selon l'une quelconque des revendications 1 à 16 ou un polynucléotide selon la revendication 17 et optionnellement un excipient pharmaceuticallement acceptable.

19. Utilisation d'un polypeptide chimérique selon l'une quelconque des revendications 1 à 16 ou un polynucléotide selon la revendication 17 pour la fabrication d'un médicament destiné à traiter le cancer.


### SEQUENCE LISTING

<110> DIATOS S.A.
   DE COUPADE, Catherine
<120> COMPOSITIONS AND METHODS FOR DELIVERING ANTI-ACTIVATED RAS ANTIBODIES
<130> 46408/PCT
<140> PCT/IB2007/001546
   <141> 2007-06-08
<150> EP 06290952.8
   <151> 2006-06-12
<160> 48
<170> PatentIn version 3.3
<210> 1
   <211> 21
   <212> PRT
   <213> Artificial
<220>
   <223> cell penetrating peptide
<400> 1
   Thr Arg Ser Ser Arg Ala Gly Leu Gln Phe Pro Val Gly Arg Val His
   1               5                   10                  15
   Arg Leu Leu Arg Lys
               20
<210> 2
   <211> 16
   <212> PRT
   <213> Artificial
<220>
   <223> cell penetrating peptide
<400> 2
   Arg Lys Lys Arg Arg Arg Glu Ser Arg Lys Lys Arg Arg Arg Glu Ser
   1               5                   10                  15
<210> 3
   <211> 17
   <212> PRT
   <213> Artificial
<220>
   <223> cell penetrating peptide
<400> 3
   Gly Arg Pro Arg Glu Ser Gly Lys Lys Arg Lys Arg Lys Arg Leu Lys
   1               5                   10                  15
   Pro
<210> 4
   <211> 15
   <212> PRT
   <213> Artificial
<220>
   <223> cell penetrating peptide
<400> 4
   Gly Lys Arg Lys Lys Lys Gly Lys Leu Gly Lys Lys Arg Asp Pro
   1               5                   10                  15
<210> 5
   <211> 17
   <212> PRT
   <213> Artificial
<220>
   <223> cell penetrating peptide
<400> 5
   Gly Lys Arg Lys Lys Lys Gly Lys Leu Gly Lys Lys Arg Pro Arg Ser
   1               5                   10                  15
   Arg
<210> 6
   <211> 18
   <212> PRT
   <213> Artificial
<220>
   <223> cell penetrating peptide
<400> 6
   Arg Lys Lys Arg Arg Arg Glu Ser Arg Arg Ala Arg Arg Ser Pro Arg
   1 5 10 15
   His Leu
<210> 7
   <211> 19
   <212> PRT
   <213> Artificial
<220>
   <223> cell penetrating peptide
<400> 7
   Ser Arg Arg Ala Arg Arg Ser Pro Arg Glu Ser Gly Lys Lys Arg Lys
   1 5 10 15
   Arg Lys Arg
<210> 8
   <211> 19
   <212> PRT
   <213> Artificial
<220>
   <223> cell penetrating peptide
<400> 8
   Val Lys Arg Gly Leu Lys Leu Arg His Val Arg Pro Arg Val Thr Arg
   1 5 10 15
   Met Asp Val
<210> 9
   <211> 18
   <212> PRT
   <213> Artificial
<220>
   <223> cell penetrating peptide
<400> 9
   Val Lys Arg Gly Leu Lys Leu Arg His Val Arg Pro Arg Val Thr Arg
   1 5 10 15
   Asp Val
<210> 10
   <211> 14
   <212> PRT
   <213> Artificial
<220>
   <223> cell penetrating peptide
<400> 10
   Ser Arg Arg Ala Arg Arg Ser Pro Arg His Leu Gly Ser Gly
   1               5                   10
<210> 11
   <211> 16
   <212> PRT
   <213> Artificial
<220>
   <223> cell penetrating peptide
<400> 11
   Leu Arg Arg Glu Arg Gln Ser Arg Leu Arg Arg Glu Arg Gln Ser Arg
   1               5                   10                  15
<210> 12
   <211> 22
   <212> PRT
   <213> Artificial
<220>
   <223> cell penetrating peptide
<400> 12
   Gly Ala Tyr Asp Leu Arg Arg Arg Glu Arg Gln Ser Arg Leu Arg Arg
   1               5                   10                  15
   Arg Glu Arg Gln Ser Arg
               20
<210> 13
   <211> 30
   <212> PRT
   <213> Artificial
<220>
   <223> cell penetrating peptide
<400> 13
   Trp Glu Ala Ala Leu Ala Glu Ala Leu Ala Glu Ala Leu Ala Glu His
   1 5 10 15
   Leu Ala Glu Ala Leu Ala Glu Ala Leu Glu Ala Leu Ala Ala
   20 25 30
<210> 14
   <211> 21
   <212> PRT
   <213> Artificial
<220>
   <223> cell penetrating peptide
<400> 14
   Lys Gly Ser Trp Tyr Ser Met Arg Lys Met Ser Met Lys Ile Arg Pro
   1 5 10 15
   Phe Phe Pro Gln Gln
   20
<210> 15
   <211> 20
   <212> PRT
   <213> Artificial
<220>
   <223> cell penetrating peptide
<400> 15
   Lys Thr Arg Tyr Tyr Ser Met Lys Lys Thr Thr Met Lys Ile Ile Pro
   1 5 10 15
   Phe Asn Arg Leu
   20
<210> 16
   <211> 20
   <212> PRT
   <213> Artificial
<220>
   <223> cell penetrating peptide
<400> 16
   Arg Gly Ala Asp Tyr Ser Leu Arg Ala Val Arg Met Lys Ile Arg Pro
   1               5                   10                  15
   Leu Val Thr Gln
               20
<210> 17
   <211> 24
   <212> PRT
   <213> Artificial
<220>
   <223> cell penetrating peptide
<400> 17
   Leu Gly Thr Tyr Thr Gln Asp Phe Asn Lys Phe His Thr Phe Pro Gln
   1               5                   10                  15
   Thr Ala Ile Gly Val Gly Ala Pro
               20
<210> 18
   <211> 12
   <212> PRT
   <213> Artificial
<220>
   <223> cell penetrating peptide
<400> 18
   Thr Ser Pro Leu Asn Ile His Asn Gly Gln Lys Leu
   1               5                   10
<210> 19
   <211> 12
   <212> PRT
   <213> Artificial
<220>
   <223> cell penetrating peptide
<400> 19
   Asn Ser Ala Ala Phe Glu Asp Leu Arg Val Leu Ser
   1               5                   10
<210> 20
   <211> 30
   <212> PRT
   <213> Artificial
<220>
   <223> cell penetrating peptide
<400> 20
   Trp Glu Ala Lys Leu Ala Lys Ala Leu Ala Lys Ala Leu Ala Lys His
   1               5                   10                  15
   Leu Ala Lys Ala Leu Ala Lys Ala Leu Lys Ala Cys Glu Ala
               20                  25                  30
<210> 21
   <211> 10
   <212> PRT
   <213> Artificial
<220>
   <223> cell penetrating peptide
<400> 21
   Val Pro Met Leu Lys Pro Met Leu Lys Glu
   1               5                   10
<210> 22
   <211> 18
   <212> PRT
   <213> Artificial
<220>
   <223> cell penetrating peptide
<400> 22
   Lys Leu Ala Leu Lys Leu Ala Leu Lys Ala Leu Lys Ala Ala Leu Lys
   1               5                   10                  15
   Leu Ala
<210> 23
   <211> 27
   <212> PRT
   <213> Artificial
<220>
   <223> cell penetrating peptide
<400> 23
   Gly Ala Leu Phe Leu Gly Phe Leu Gly Ala Ala Gly Ser Thr Met Gly
   1 5 10 15
   Ala Trp Ser Gln Pro Lys Lys Lys Arg Lys Val
   20 25
<210> 24
   <211> 16
   <212> PRT
   <213> Artificial
<220>
   <223> cell penetrating peptide
<400> 24
   Ala Ala Val Ala Leu Leu Pro Ala Val Leu Leu Ala Leu Leu Ala Pro
   1 5 10 15
<210> 25
   <211> 9
   <212> PRT
   <213> Artificial
<220>
   <223> cell penetrating peptide
<400> 25
   Val Gln Arg Lys Arg Gln Lys Leu Met
   1 5
<210> 26
   <211> 21
   <212> PRT
   <213> Artificial
<220>
   <223> cell penetrating peptide
<400> 26
   Lys Glu Thr Trp Trp Glu Thr Trp Trp Thr Glu Trp Ser Gln Pro Lys
   1 5 10 15
   Lys Lys Arg Lys Val
   20
<210> 27
   <211> 15
   <212> PRT
   <213> Artificial
<220>
   <223> cell penetrating peptide
<400> 27
   Ser Asp Leu Trp Glu Met Met Met Val Ser Leu Ala Cys Gln Tyr
   1 5 10 15
<210> 28
   <211> 16
   <212> PRT
   <213> Artificial
<220>
   <223> cell penetrating peptide
<400> 28
   Arg Gln Ile Lys Ile Trp Phe Gln Asn Arg Arg Met Lys Trp Lys Lys
   1 5 10 15
<210> 29
   <211> 7
   <212> PRT
   <213> Artificial
<220>
   <223> cell penetrating peptide
<400> 29
   Arg Arg Met Lys Trp Lys Lys
   1 5
<210> 30
   <211> 7
   <212> PRT
   <213> Artificial
<220>
   <223> cell penetrating peptide
<400> 30
   Arg Arg Arg Arg Arg Arg Arg
   1 5
<210> 31
   <211> 9
   <212> PRT
   <213> Artificial
<220>
   <223> cell penetrating peptide
<400> 31
   Arg Arg Arg Arg Arg Arg Arg Arg Arg
   1               5
<210> 32
   <211> 16
   <212> PRT
   <213> Artificial
<220>
   <223> cell penetrating peptide
<400> 32
   Arg Val Ile Arg Val Trp Phe Gln Asn Lys Arg Cys Lys Asp Lys Lys
   1               5                   10                  15
<210> 33
   <211> 28
   <212> PRT
   <213> Artificial
<220>
   <223> cell penetrating peptide
<400> 33
   Met Ala Asn Leu Gly Tyr Trp Leu Leu Ala Leu Phe Val Thr Met Trp
   1               5                   10                  15
   Thr Asp Val Gly Leu Cys Lys Lys Arg Pro Lys Pro
               20                  25
<210> 34
   <211> 18
   <212> PRT
   <213> Artificial
<220>
   <223> cell penetrating peptide
<400> 34
   Leu Leu Ile Ile Leu Arg Arg Arg Ile Arg Lys Gln Ala His Ala His
   1               5                   10                  15
   Ser Lys
<210> 35
   <211> 18
   <212> PRT
   <213> Artificial
<220>
   <223> cell penetrating peptide
<400> 35
   Val Arg Leu Pro Pro Pro Val Arg Leu Pro Pro Pro Val Arg Leu Pro
   1               5                   10                  15
   Pro Pro
<210> 36
   <211> 7
   <212> PRT
   <213> Artificial
<220>
   <223> cell penetrating peptide
<400> 36
   Pro Lys Lys Lys Arg Lys Val
   1               5
<210> 37
   <211> 18
   <212> PRT
   <213> Artificial
<220>
   <223> cell penetrating peptide
<400> 37
   Arg Gly Gly Arg Leu Ser Tyr Ser Arg Arg Arg Phe Ser Thr Ser Thr
   1               5                   10                  15
   Gly Arg
<210> 38
   <211> 10
   <212> PRT
   <213> Artificial
<220>
   <223> cell penetrating peptide
<400> 38
   Arg Arg Leu Ser Tyr Ser Arg Arg Arg Phe
   1               5                   10
<210> 39
   <211> 17
   <212> PRT
   <213> Artificial
<220>
   <223> cell penetrating peptide
<400> 39
   Ala Trp Ser Phe Arg Val Ser Tyr Arg Gly Ile Ser Tyr Arg Arg Ser
   1               5                   10                  15
   Arg
<210> 40
   <211> 14
   <212> PRT
   <213> Artificial
<220>
   <223> cell penetrating peptide
<400> 40
   Tyr Gly Arg Lys Lys Arg Arg Gln Arg Arg Arg Pro Pro Gln
   1               5                   10
<210> 41
   <211> 11
   <212> PRT
   <213> Artificial
<220>
   <223> cell penetrating peptide
<400> 41
   Tyr Gly Arg Lys Lys Arg Arg Gln Arg Arg Arg
   1               5                   10
<210> 42
   <211> 9
   <212> PRT
   <213> Artificial
<220>
   <223> cell penetrating peptide
<400> 42
   Arg Lys Lys Arg Arg Gln Arg Arg Arg
   1               5
<210> 43
   <211> 27
   <212> PRT
   <213> Artificial
<220>
   <223> cell penetrating peptide
<400> 43
   Gly Trp Thr Leu Asn Ser Ala Gly Tyr Leu Leu Gly Lys Ile Asn Leu
   1               5                   10                  15
   Lys Ala Leu Ala Ala Leu Ala Lys Lys Ile Leu
               20                  25
<210> 44
   <211> 21
   <212> PRT
   <213> Artificial
<220>
   <223> cell penetrating peptide
<400> 44
   Ala Gly Tyr Leu Leu Gly Lys Ile Asn Leu Lys Ala Leu Ala Ala Leu
   1               5                   10                  15
   Ala Lys Lys Ile Leu
               20
<210> 45
   <211> 12
   <212> PRT
   <213> Artificial
<220>
   <223> cell penetrating peptide
<400> 45
   Gly Trp Thr Leu Asn Ser Ala Gly Tyr Leu Leu Gly
   1               5                   10
<210> 46
   <211> 14
   <212> PRT
   <213> Artificial
<220>
   <223> cell penetrating peptide
<400> 46
   Ile Asn Leu Lys Ala Leu Ala Ala Leu Ala Lys Lys Ile Leu
   1               5                   10
<210> 47
   <211> 34
   <212> PRT
   <213> Artificial
<220>
   <223> cell penetrating peptide
<400> 47
   Asp Ala Ala Thr Ala Thr Arg Gly Arg Ser Ala Ala Ser Arg Pro Thr
   1               5                   10                  15
   Glu Arg Pro Arg Ala Pro Ala Arg Ser Ala Ser Arg Pro Arg Arg Pro
               20                  25                  30
   Val Asp
<210> 48
   <211> 26
   <212> PRT
   <213> Artificial
<220>
   <223> cell penetrating peptide
<400> 48
   Asp Pro Lys Gly Asp Pro Lys Gly Val Thr Val Thr Val Thr Val Thr
   1               5                   10                  15
   Val Thr Gly Lys Gly Asp Pro Lys Pro Asp
               20                  25